# EUROPÄISCHE PATENTSCHRIFT

(11) **EP 0 321 973 B1**
(45) Veröffentlichungstag und Bekanntmachung des Hinweises auf die Patenterteilung: **03.11.1993**
(21) Anmeldenummer: 88121480.3
(22) Anmeldetag: 22.12.1988
(51) Int. Cl.: C12N 15/41, C07H 21/04, C12Q 1/37, C12Q 1/68

(54) **Expression der viral kodierten Protease P2A des HRV2**
Expression of the virally encoded protease P2A of HRV2
Expression de la protéase P2A de HRV2 codée par un virus

(30) Priorität: 23.12.1987 DE 3743848; 23.07.1988 DE 3825118
(43) Veröffentlichungstag der Anmeldung: 28.06.1989
(73) Patentinhaber: BOEHRINGER INGELHEIM INTERNATIONAL GmbH, 55218 Ingelheim am Rhein (DE)
(72) Erfinder: Sommergruber, Wolfgang, Dr., A-1130 Wien (AT); Fessl, Friederike, A-2480 Perchtolsdorf (AT); Küchler, Ernst, Prof. Dr., A-1190 Wien (AT); Blaas, Dieter, Dipl.-Ing. Dr., A-1190 Wien (AT); Skern, Timothy, Dr., A-1160 Wien (AT); Zorn, Manfred, A-1140 Wien (AT); Düchler, Markus, A-1040 Wien (AT); Kowalski, Heinrich, A-1170 Wien (AT); Volkmann, Peter, Dr., A-1238 Wien (AT); Maurer-Fogy, Ingrid, Dr., A-1238 Wien (AT); Pallai, Peter, Dr., Brookfield, CT 06804 (US); Merluzzi, Vincent, Dr., Holmes, NY 12531 (US)

(56) Entgegenhaltungen:
- PROCEEDINGS OF THE NATIONAL ACADEMY OF SCIENCES OF THE USA, Band 83, Nr. 15, August 1986, Seiten 5392-5396, Washington, US; L.A. IVANOFF et al.: "Expression and site-specific mutagenesis of the poliovirus 3C protease in Escherichia coli"
- JOURNAL OF VIROLOGY, Band 57, 1986, Seiten 1084-1093, American Society for Microbiology; G. WERNER et al.: "Molecular Cloning and Sequence Determination of the Genomic Regions Encoding Protease and Genome-Linked Protein of Three Picornaviruses"
- NUCLEIC ACIDS RESEARCH, Band 13, Nr. 6, 1985, Seiten 2111-2116; T. SKERN et al.: "Human rhinovirus 2: complete nucleotide sequence and proteolytic processing signals in the capsid protein region"
- J. GEN. VIROLOGY, Band 68, 1987, Seiten 315-323, SGM, GB; T. SKERN et al.: "A Neutralizing Epitope on Human Rhinovirus Type 2 Includes Amino Acid Residues between 153 and 164 of Virus Capsid Protein VP2"
- From Genes to clones, VCH, 1987, p. 137-138

## Beschreibung

Gegenstand der vorliegenden Erfindung sind DNA-Moleküle, die für Fusionsproteine aus enzymatisch aktiven Anteilen und von diesen abspaltbaren Polypeptidanteilen kodieren, Expressionssysteme, die diese DNA-Moleküle enthalten sowie die Verwendung dieser als Testsysteme für Inhibitoren viraler Proteasen.

Rhinoviren sind ss(+)RNA - Viren und repräsentieren eine Gattung innerhalb der Picornaviridae (Cooper, P. D et al., 1978, Intervirology 10, 165 - 180; MacNaughton, M. R., 1982, Current Top. Microbiol. Immunol. 97, 1 - 26). Sie sind weit verbreitet, befallen den oberen respiratorischen Trakt des Menschen und verursachen akute Infektionen, die zu Schnupfen, Husten, Heiserkeit etc. führen und allgemein als Erkältungen bezeichnet werden (Stott, E. J. und Killington, R. A., 1972, Ann. Rev. Microbiol. 26, 503 - 524). Infektionen durch Rhinoviren zählen zu den häufigsten Erkrankungen des Menschen. Die Krankheit verläuft zwar meist harmlos, dennoch kommt es -bedingt durch eine vorübergehende Schwächung des Organismus- zu Sekundärinfektionen durch andere Viren oder Bakterien, die dann unter Umständen schwere Erkrankungen zur Folge haben. Von den insgesamt ca. 115 verschiedenen, bekannten Serotypen von humanen Rhinoviren sind bis jetzt 3 Serotypen kloniert und komplett sequenziert worden: Deutsche Patentanmeldung P 35 05 148.5; Skern; T. et al., 1985; Nucleic Acids Res. 13, 2111-2126; Düchler, M. et al., 1987, Proc. Natl. Acad. Sci. USA 84, 2605 - 2609; Stanway, G. et al., 1984, Nucleic Acids Res. 12, 7859 - 7877; Callahan, P. L. et al., 1985, Proc. Natl. Acad. Sci. USA 82, 732 - 736).

Ein Vergleich der Aminosäuresequenzen der einzelnen Proteine zeigt, daß die viralen Enzyme in besonderem Maße konserviert sind. So beträgt etwa die Homologie zwischen der Protease P2A von HRV89 und HRV2 85%; bei der Protease P3C sind 75% der Aminosäuren identisch (Düchler, M. et al., 1987). Diese Werte liegen wesentlich über den durchschnittlich im Gesamtprotein beobachteten Prozentsätzen. Man kann daher davon ausgehen, daß gerade die viralen Enzyme in der Evolution besonders gut konserviert sind und in ihren Eigenschaften bei verschiedenen Rhinoviren sehr ähnlich sind.
Kaum ein anderes virales System ist bei seiner Regulation des Infektionsablaufes dermaßen von einer kontrolliert limitierten Proteolyse abhängig, wie das der Picornaviridae. Die genomische einzelsträngige (+)RNA der Rhinoviren wird kurz nach der Infektion durch Abspaltung des an das 5′ Ende gebundenen Oligopeptids VPg modifiziert und dient als mRNA für die Synthese eines Polyproteins, das den gesamten durchgehenden Leserahmen der Nukleinsäuresequenz umfaßt (Butterworth, B. E., 1973, Virology 56, 439 - 453; Mc Lean, C. und Rueckert, R. R., 1973, J. Virol. 11, 341 - 344; Mc Lean, C. et al., 1976, J. Virol. 19, 903 - 914). Die reifen viralen Proteine entstehen ausschließlich durch proteolytische Spaltung aus diesem Polyprotein, wobei die dabei wirksamen Proteasen selbst Bestandteil dieses Polyproteins sind. Der erste Schritt bei diesem Prozessieren ist die Abspaltung der Vorstufe der Hüllenproteine, die durch die Protease P2A vorgenommen wird. In der Reihenfolge der Gene liegt die Sequenz der Protease P2A unmittelbar hinter dem für die Hüllenproteine kodierenden Abschnitt. P2A ist somit aufgrund ihrer Lokalisation im Polyprotein die erste nachweisbare enzymatische Funktion des Virus.
Sie spaltet sich gewissermaßen selbst vom Precursor der Hüllenproteine ab und ist für die Trennung des Kapsidprecursors P1 vom Rest des Polyproteins verantwortlich. Die Trennung der Hüllenproteinregion von dem für die Replikation verantwortlichen Abschnitt findet bereits während der Translation des Polyproteins statt.

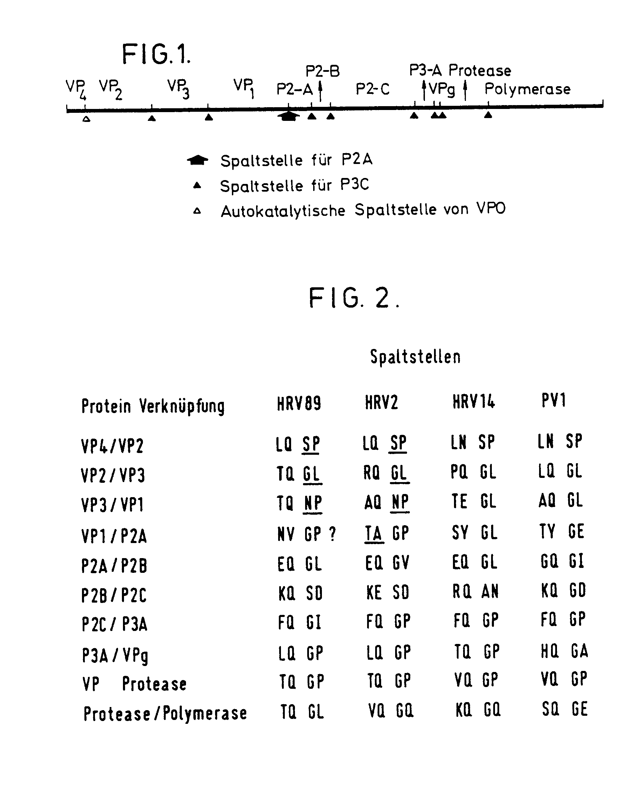

Dieser Schritt ist für den weiteren Ablauf der viralen Infektion essentiell.
Vom Poliovirussystem weiß man, daß wahrscheinlich alle an dieser Reifungsspaltung beteiligten Enzyme viral kodiert sind (Toyoda, H. et al., 1986, Cell, 45, 761-770). Im Poliovirus findet man drei Typen von Spaltsignalen (Fig. 1); die am meisten verwendeten Q-G Stellen, welche von der viralen Protease P3C erkannt werden, und die Y-G Stelle, welche von P2A als Erkennungssignal verwendet wird. Zunächst rückte die Protease P3C in den Mittelpunkt des Interesses bei der Aufklärung des proteolytischen Processings von Picornaviren. Schon sehr früh konnte eine der P3C äquivalente proteolytische Aktivität in EMC beschrieben werden (Pelham, H. R. B. , 1978, Eur. J. Biochem. 85, 457 - 461; Palmenberg A. C. et al., 1979, J. Virol. 32, 770 - 778). Im Laufe weiterer Untersuchungen stellte sich heraus, daß das Leaderpeptid (L) von Cardio- (z. B. EMCV) und Aphtoviren (z. B. FMDV), welches bei Rhino- und Enteroviren nicht vorhanden ist, am proteolytischen Processing von EMCV beteiligt ist (Palmenberg, A. C., 1987, J. Cell. Biochem. 33, 1191-1198). In weiterer Folge konnte durch die Isolierung von Polio P3C und mit Hilfe von immunologischen Methoden gezeigt werden, daß P3C sich selbst autokatalytisch aus dem Polyprotein herausschneidet, um dann in "trans" alle potentiellen Q-G Spaltstellen anzugreifen.

Der Einsatz von rekombinanten Systemen, die unter anderem die P3C-Region repräsentierten, ermöglichte die Expression von P3C einiger Entero- und Rhinoviren (Werner, G. et al., 1986, J. Virol. 57, 1084 - 1093) und die genaue Charakterisierung der P3C von Polio (Hanecak, R. et al., 1984, Cell 37, 1037 - 1073; Korant, B. D. und Towatari, T., 1986, Biomed. Biochim. Acta 45, 1529 - 1535) sowie der äquivalenten proteolytischen Funktion in FMDV (Klump, W. et al., 1984, Proc. Natl. Acad. Sci. USA 81, 3351 - 3355; Burroughs, J. N. et al., 1984, J. Virol. 50, 878 - 883). Durch in vitro Mutagenesestudien konnte nachgewiesen werden, daß der Austausch der hoch konservierten Aminosäuren Cystein (Positionsnummer 147) und Histidin (Positionsnummer 161) in P3C von Poliovirus zu einem inaktiven Enzym führt, während die Mutation des nicht konservierten Cysteins (Positionsnummer 153) keinen nennenswerten Einfluß auf die proteolytische Aktivität von Polio-P3C hat. Diese durch Oligonukleotide bewirkte Mutagenese von rekombinanten P3C und die zusätzlich durchgeführten Inhibitorstudien lassen den Schluß zu, daß Polio-P3C zu der Klasse der Cysteinproteasen gehört (Ivanoff, L. A. et al., 1986, Proc. Natl. Acad. Sci. USA 83, 5392 - 5396). Ebenfalls durch in vitro Mutagenese von Polio-P3C (und zwar durch Austausch des konservierten Valin gegen Alanin in Position 54 der Protease) konnte gezeigt werden, daß diese Mutation in einer "full size" cDNA von Polio nach Transfektion in COS 1 Zellen zu einem Polymerase-defizienten Virus führt (Dewalt, P. G. und Semler, B. L., 1987,J. Virol. 61, 2162 - 2170).

Antikörper, die gegen Polio-P3C entwickelt wurden, unterbanden zwar eindeutig sämtliche an Q-G durchgeführten Spaltungen, nicht aber die Spaltung zwischen Y-G (Hanecak, R. et al., 1982, Proc. Natl. Acad. Sci. USA 79, 3973 - 3977). Diese Beobachtung führte zum Schluß, daß das proteolytische Processing an Y - G Stellen einer eigenen Protease bedarf. Der Sitz dieser zweiten proteolytischen Aktivität konnte in Poliovirus eindeutig P2A zugeordnet werden. Interessant war der Befund, daß P2A eine alternative Spaltung in der Protease-Polymeraseregion (3CD) durchführt, welche ebenfalls an einer Y - G Stelle stattfindet. Dieser Spaltung dürfte aber keine biologische Bedeutung während der Virusreplikation zukommen (Toyoda, H. et al., loc. cit.).
Da es während der Infektion mit Poliovirus in HeLa-Zellen sehr rasch zu einem Abschalten der Wirtsproteinsynthese kommt, die Translation der Poliovirus-RNA jedoch unbehindert ablaufen kann, nahm man an, daß ein oder mehrere Regulationsfaktoren der Translation während der Infektion verändert werden. Tatsächlich zeigen ältere Befunde, daß der eukaryontische Initiationsfaktor 4F durch proteolytische Spaltung der p220 Komponente während der Poliovirusinfektion in HeLa-Zellen verändert wird (Etchison, D. et al., 1984, J. Virol. 51, 832 - 837; Etchison, D. et al., 1982, J. Biol. Chem. 257, 14806 - 14810). In der Folge konnte gezeigt werden, daß P2A indirekt für diese Modifikation von p220 in infizierten Zellen verantwortlich ist (Kräusslich, H. G. et al., 1987, J. Virol. 61, 2711 - 2718).
Die Frage nach der Transaktivität der beiden Proteasen P3C und P2A konnte im Poliovirussystem insofern positiv beantwortet werden, als in vitro exprimierte Poliovirus-Polypeptidvorstufen, die die proteolytischen Erkennungsequenzen enthielten, durch exogene P3C bzw. P2A Proteasen prozessiert werden konnten (Nicklin, M. J. H. et al., 1987, Proc. Natl. Acad. Sci. USA 84, 4002 - 4006).

Sehr interessant sind auch die Befunde, daß zwei den picornaviralen Proteasen P3C und P2A ähnliche Proteine in dem pflanzenviralen System der Comoviridae (Cowpea Mosaic Virus) gefunden wurden (Garcia, J. A. et al., 1987, Virology 159, 67 - 75; Verver, J. et al., 1987, EMBO 6, 549 - 554). Diese beiden viralen Proteine sind am proteolytischen Processing der beiden durch zwei getrennt verpackte ss (+)RNA-Moleküle (B und M RNA) kodierten Polyproteine beteiligt, wobei eine große Ähnlichkeit der beiden Cowpea mosaic-Virus-Proteasen in Sequenz und Spaltspezifität zu den Picornaviren festzustellen ist. Diese bemerkenswerte Homologie von nicht strukturellen Proteinen zwischen Picorna- und Comoviren weist nicht nur auf eine genetische Verwandtschaft zwischen diesen beiden Virusfamilien hin, sondern zeigt auch auf, wie essentiell das virale proteolytische Processing für diese beiden Virusfamilien ist.
Die dritte Art der viralen Reifungspaltung, nämlich die von VP0 (Vorläuferprotein von VP2 und VP4) konnte bei Mengo und Rhinovirus mit Hilfe von Röntgenstrukturdaten beschrieben werden. Dieses letzte proteolytische Ereignis bei der viralen Maturation scheint auf einem ungewöhnlichen autokatalytischen Serinprotease Typ zu beruhen, bei welchem basische Gruppen der viralen RNA an der Ausbildung des katalytischen Zentrums beteiligt sind, wobei diese basischen Gruppen als Protonenakzeptor fungieren (Arnold, E. et al., 1987, Proc. Natl. Acad. Sci. USA 84, 21 - 25).

Die Spaltstellenspezifität der viralen Proteasen wurde im Poliovirussystem durch N-terminales Sequenzieren der meisten Poliovirusproteine ermittelt (Pallansch, M. A. et al., 1984, J. Virol. 49, 873 - 880). Durch das Klonieren und Sequenzieren von HRV2 (Skern, T. et al., 1985 Nucleic Acids Res. 13, 2111-2126) konnten an Hand von Sequenzvergleichen mit Poliovirus und HRV14 die meisten Spaltstellen abgeleitet werden. Außerdem konnte die Lage der Schnittstellen zwischen VP4/VP2, VP2/VP3 sowie VP3/VP1 durch N-terminales Sequenzieren von VP2, VP3 und VP1 bestimmt werden. Das Spaltsignal zwischen VP1 und P2A wurde teilweise durch C-terminales Sequenzieren von VP1 bestimmt (Kowalski, H. et al., 1987, J. Gen. Virol. 86, 3197 - 3200). So konnten in HRV2 fünf verschiedene Spaltsignale gefunden werden: Q-S, Q-G, Q-N, A-G und E-S (Fig. 2).

Cysteinproteasen sind in der Natur weit verbreitet (z. B. Papain, Cathepsin B, H und S) und ihre Charakterisierung und Inhibierung ist von großem wissenschaftlichen und therapeutischen Wert (zur Übersicht siehe Turk, V., 1986, Cysteine Proteinases and their Inhibitors, Walter de Gruyter; Barrett, A. J. und Salvesen, G., 1986, Proteinase Inhibitors, Elsevier). Auch im picornaviralen System sind derzeit verschiedenste anorganische und organische Verbindungen, sowie Peptidderivate und Proteine bekannt, die eine inhibitorische Wirkung auf das proteolytische Processing dieser Viren besitzen. Der Effekt dieser Substanzen beruht auf der direkten Wechselwirkung mit den Proteasen (Kettner, C. A. et al., 1987, US Patent Nr.: 4,652,552; Korant, B. D. et al., 1986, J. Cell. Biochem. 32, 91 -95) und/oder auf dem indirekten Weg der Wechselwirkung mit Substraten dieser Proteasen (Geist, F. C., et al., 1987, Antimicrob. Agents Chemother. 31, 622 - 624; Perrin, D. D. und Stünzi, H., 1984, Viral Chemotherapy 1, 288 - 189). Das Problem bei den meisten dieser Substanzen ist die relativ hohe Konzentration, die zur Inhibierung nötig ist und die zum Teil große Toxizität dieser Verbindungen.

Wie bereits dargelegt wurde, ist der Infektionsablauf durch Picornaviren entscheidend von den viralen Enzymen abhängig. Da gerade diese Enzyme besonders gut konserviert und in ihren Eigenschaften bei verschiedenen Rhinoviren sehr ähnlich sind, bieten sie sich geradezu als Ziel eines chemotherapeutischen Eingriffs an. Besonders bevorzugt ist hierbei das virale Enzym P2A. Der chemotherapeutische Ansatz ist vorzugsweise die Inhibierung der enzymatischen Aktivität durch beispielsweise spezifische Inhibitoren.

Inhibiert man die erste proteolytische Aktivität, die P2A-Aktivität, so unterbindet man jeden weiteren Reifungsprozeß des viralen Systems. Auf Grund der ausgeprägten Homologie der P2A-Region von HRV2 nicht nur zu anderen Rhinoviren, sondern auch zu Vertretern anderer Gruppen der Picornaviridae, ist es durchaus denkbar, daß ein Inhibitor gegen HRV2-P2A auch auf andere Picornaviren anwendbar ist.

Aufgabe der vorliegenden Erfindung war es daher, ein System bereitzustellen, mit dem es möglich ist, potentielle Inhibitoren für den viralen Reifungsprozeß auszutesten.

Da P2A "in statu nascendi" aktiv ist und selbst Bestandteil des Substrates ist, mußte ein geeignetes "Substratenzym" konstruiert werden, das es ermöglicht die P2A-Aktivität anhand ihrer Spaltprodukte zu verfolgen. Ferner erkennt P2A nur eine Spaltstelle im Polyprotein. Gerade diese Eigenschaft macht die virale Funktion zum geeigneten Ziel eines therapeutischen Angriffs. Unter diesem Gesichtspunkt wurde in der vorliegenden Erfindung ein Expressionssystem von HRV2-P2A entwickelt, um P2A näher zu charakterisieren und eine geeignete Methode zur Auffindung von Inhibitoren gegen P2A zu etablieren.

Dieses Expressionssystem, bestehend aus einem Plasmidanteil und einem Insert, mußte in der Lage sein, ein als Substrat dienendes virales Polypeptid, das gleichzeitig auch P2A-Protease-Aktivität aufweist, zu produzieren. Erfindungsgemäß enthält ein solches System als Insert ein DNA-Molekül, das für ein Fusionsprotein aus einem enzymatisch aktiven Anteil und einem von diesem abspaltbaren Polypeptidanteil kodiert. Der enzymatisch aktive Anteil ist dabei eine virale Protease, bevorzugt die virale Protease HRV2-P2A. Der abspaltbare Polypeptidanteil ist ein virales Protein, bevorzugt ein virales Protein VP1, besonders bevorzugt das virale Protein HRV2-VP1, insbesondere (VP3)-VP1 von HRV2 oder Teilen von diesen.

Ein Beispiel für ein solches System enthält als Insert vorzugsweise die HRV2 Sequenz von 2145-3698 im richtigen Leserahmen. Dieses Insert kann in jeden beliebigen Expressionsvektor eingefügt werden, der in der Lage ist, in einem, mit diesem Vektor transformierten geeigneten Wirtsorganismus, das Substrat effektiv zu produzieren. Ein solcher Expressionsvektor enthält vorzugsweise eine ribosomale Bindungsstelle, eine kodierende Region für einen die Stabilität des exprimierten, von dem Insert kodierten Fusionsproteins in der Zelle erhöhenden Fusionsanteils, einen das Fusionsprotein kontrollierenden Promotor, eine Polylinkerregion in drei verschiedenen Leserahmen, vorzugsweise mit Schnittstellen für Restriktionsenzyme eine "ori"-Region und einen Selektionsmarker.

Besonders bevorzugt ist:
- die prokaryontische ribosomale Bindungsstelle
- einen Teil der kodierenden Region für die ersten 98 N-terminalen Aminosäuren der MS2-Polymerase; dieser Fusionsanteil weist hydrophobe und basische Aminosäuren auf und bewirkt die Herabsetzung der Löslichkeit des Fusionsproteins und erhöht die Stabilität des exprimierten Produktes in der Zelle.
- das Fusionsprotein steht unter der Kontrolle des linken lambda-Promotors
- eine kleine Polylinkerregion in 3 verschiedenen Leserahmen (pExa,b und c) mit Schnittstellen für EcoRI, BamHI, HindIII, PstI, BglII und XbaI ermöglicht das Einsetzen von geeigneten DNA-Fragmenten in Phase hinter dem Fusionsproteinanteil
- die "ori"- und die Ampicillin-resistenz-region von pBR322.

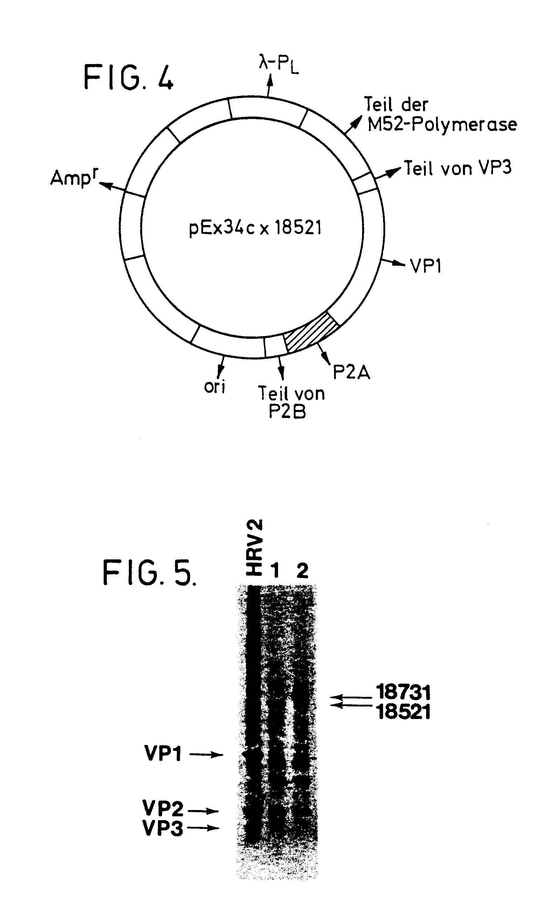

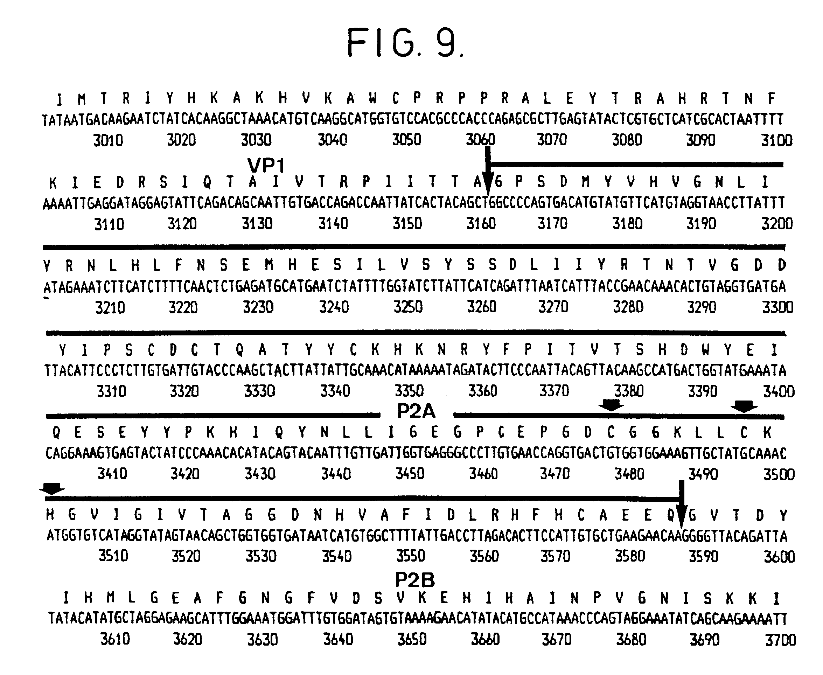

Beispiele solcher Vektoren sind Derivate des Plasmides pPLc-24 (E. Remault, P. Stanssens und W. Fiers; 1981, Gene 15, 81-93), die bereits durch K. Strebel et al. 1986, J. Virol. 57, 983-991 als Derivate in Form von pEx-Vektoren bekannt wurden. Der in der vorliegenden Erfindung verwendete pEx34c Expressionsvektor ist ein solches Derivat. In diesen Vektor wurde das oben erwähnte Insert als EcoRI/HindIII Fragment eingefügt. Man erhielt pEx34c x 18521 (Fig. 4). Zur Durchführung von Expressionsstudien wurde darüberhinaus noch ein inaktives Enzymsubstrat für P2A benötigt, das beispielsweise durch Deletionsmutation erhalten wurde. Eine solche Deletionsmutante des pEx34c x 18521 wurde pEx34c x 18731 genannt. Diese Mutante endet bei Nukleotidnummer 3321 und besitzt daher das vermutete aktive Zentrum der P2A nicht mehr (Fig. 9).

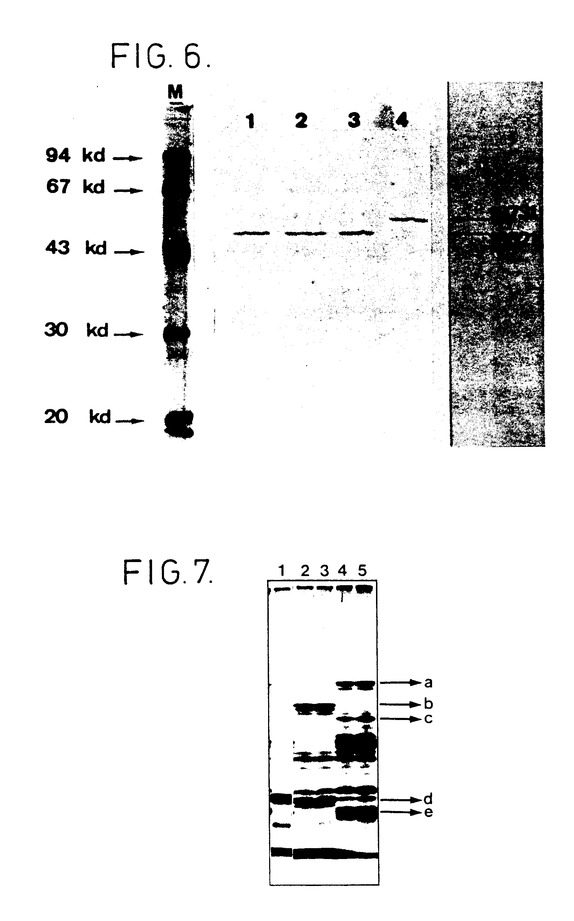

Die antigenische Spezifität der exprimierten Substrate von pEx34c x 18521 bzw. pEx34c x 18731 wurde mit Hilfe eines polyklonalen Serums gegen VP1, das integraler Bestandteil beider Plasmide ist, durch einen Westernblot nachgewiesen (Fig. 6).

Um die Eignung dieses Systems zur Austestung möglicher Inhibitoren zu demonstrieren, wurden die beiden Plasmide in einem bakteriellen, zellfreien System, beispielsweise dem "Procaryotic DNA directed Translation kit" von Amersham in vitro zur Expression gebracht. Das Ergebnis gibt Fig. 7 wieder. Sie zeigt, daß sich ein solches in vitro System hervorragend eignet, um Inhibitoren gegen P2A auszutesten.

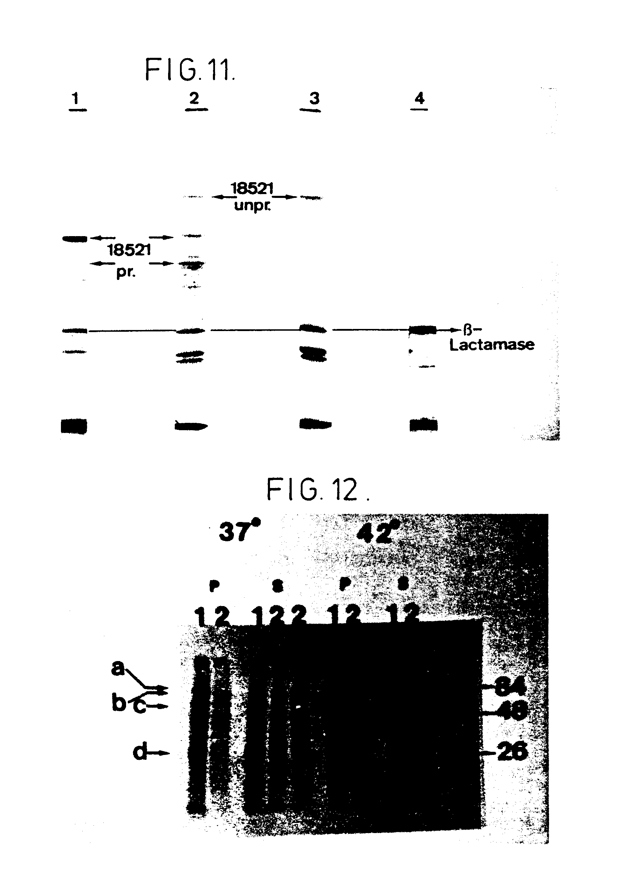

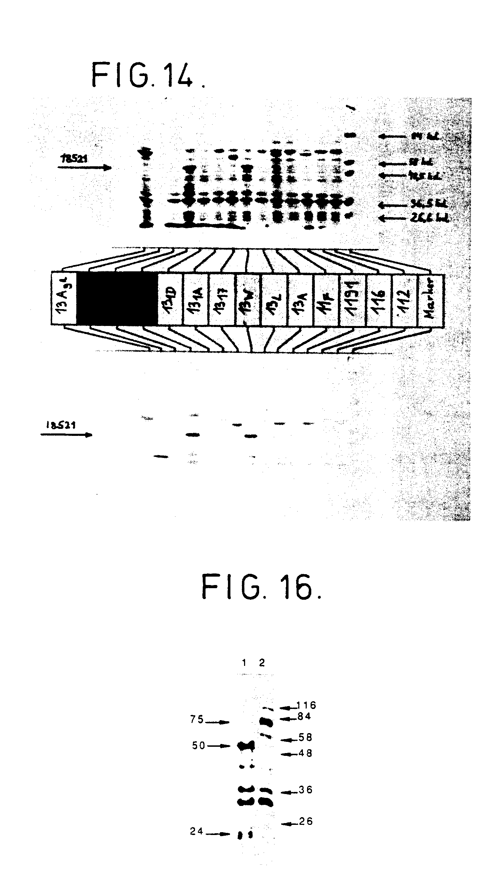

Um weitere Informationen über das katalytische Zentrum der viralen Protease P2A zu erhalten, wurden Mutagenese Studien am vermuteten aktiven Zentrum vorgenommen. Da es sich um eine SH-Protease handeln, dürfte (Cys...His im aktiven Zentrum), wurden beide in Frage kommenden Cysteine in Position 106 bzw. 112 gegen Phenylalanin durch Oligonukleotid-Mutagenese ausgetauscht. Die so mutierten Plasmide wurden im prokaryotischen in vitro Translationssystem zur Expression gebracht. Dabei zeigte sich, daß das Plasmid, in dem das Cystein an Position 112, in unmittelbarer Nähe zum Histidinrest ausgetauscht wurde, eine erhöhte P2A-Aktivität aufwies. Die Mutation an Position 106 führte zu einer Inhibierung der proteolytischen Aktivität.
Durch in vivo Markierung von HRV2-P2A und Vergleich der Expressionsprodukte von pEx34c x 18521 mit denen einer weiteren Deletionsmutante konnten essentielle Teile des C-Terminus von P2A weiter abgesichert werden. Diese Mutante, die mit der für HRV2 kodierenden Region 18 Aminosäuren vor dem Carboxyterminus von P2A endet, wurde 13L genannt (Fig. 14). Sie wurde ebenfalls durch Einwirkung der Exonuklease Bal3l hergestellt. Sowohl pEx34c x 18521 als auch 13L wurden in E.coli hochgezüchtet und in vivo mit S-35 Methionin markiert. Die Expressionsprodukte wurden autoradiographiert (Fig. 12). Die vom Expressionssystem 18521 herrührende spezifische Bande, die P2A plus einen Teil von P2B repräsentiert, ist im Expressionssystem der Deletionsmutante 13L nicht zu erkennen.

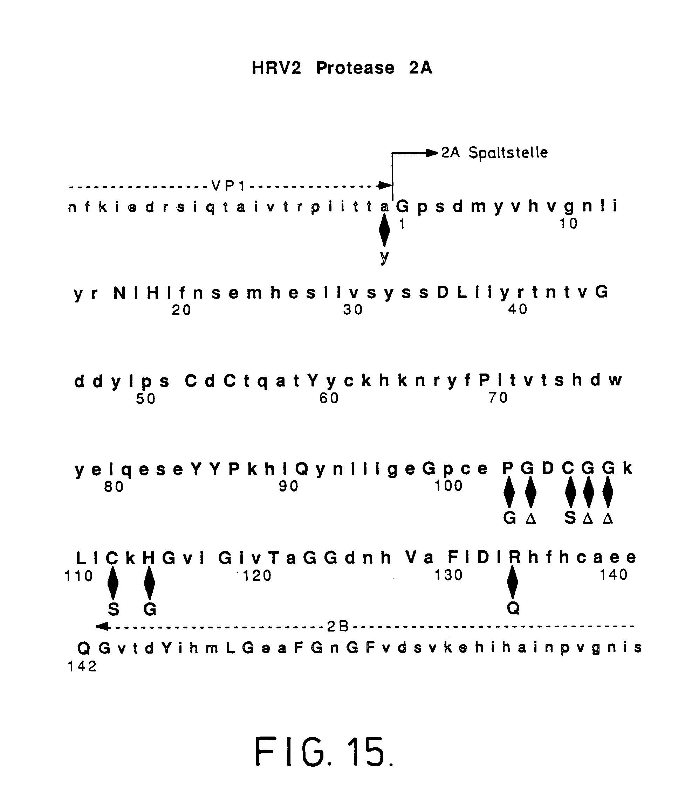

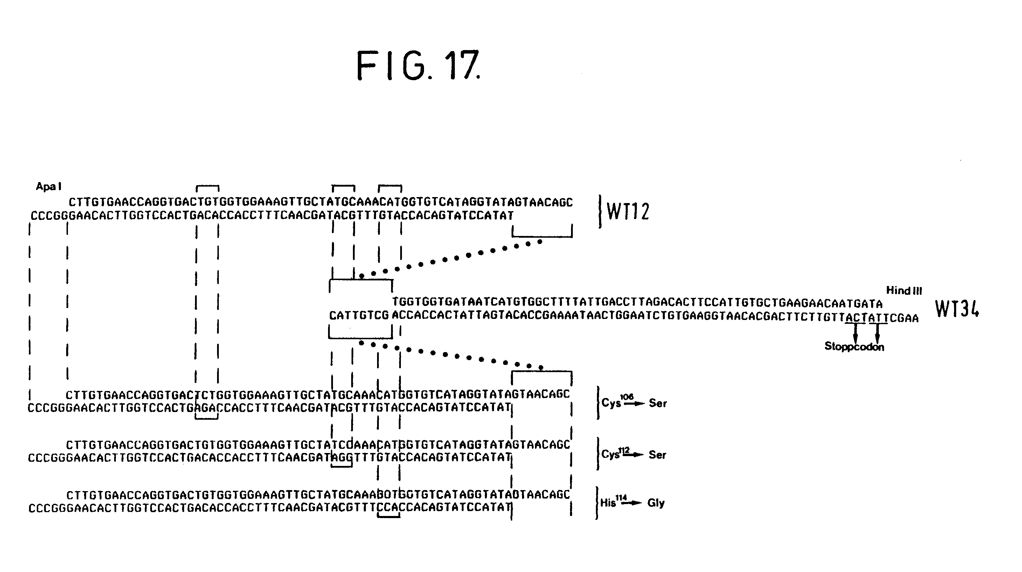

Die Deletionsstudien im C-terminalen Bereich der Protease 2A (siehe Beispiel 5) zeigten, daß dieser Bereich von 2A um mindestens 6 Aminosäuren verkürzt werden kann, ohne das proteolytische Processing zu beeinflussen (siehe Fig. 14). Die Deletion zwischen der zehnten und sechsten Aminosäure vom C-Terminus von 2A (entspricht der 137. und 133. Aminosäure in Fig. 15) führt offensichtlich zur Zerstörung eines essentiellen Abschnitts von 2A. Allen weiteren Mutanten, welche eine Deletion von mehr als 10 Aminosäuren am C-Terminus von 2A aufweisen, fehlt ebenfalls die Fähigkeit die VP1/2A Spaltstelle proteolytisch zu prozessieren (siehe Beispiel 5, Fig. 14). Es müssen daher zwischen der 6. und 10. Aminosäure vom C-Terminus essentielle Reste vorhanden sein, die für die katalytische Aktivität unentbehrlich sind. Betrachtet man die Aminosäuresequenz von 2A und ihre innerhalb der Rhino-, Polio- und Coxsackieviren hochkonservierten Aminosäuren (siehe Fig. 15), so erkennt man, daß zwischen der 6. und 10. Aminosäure vom C-Terminus von 2A nur das Arginin in Position 134 konserviert ist. Um nun herauszufinden, ob dieser Rest eine fundamentale Bedeutung in der Katalyse besitzt, wurde das Arginin 134 durch in vitro Mutagenese in ein Glutamin umgewandelt. Der mutierte Expressionsvektor pEx18521[Arg134-->Gln] wurde, wie in Beispiel 2 beschrieben, zur Expression gebracht und das Muster der Expressionsprodukte auf einem Proteingel und mit Hilfe eines Western blot analysiert (siehe Fig. 16). Eindeutig konnte die Bedeutung des Arg 134 nachgewiesen werden: Die Mutation des Arginin 134 zu Glutamin führt zum Verschwinden der proteolytischen Aktivität; es wird ausschließlich das unprozessierte 75K Protein gebildet.

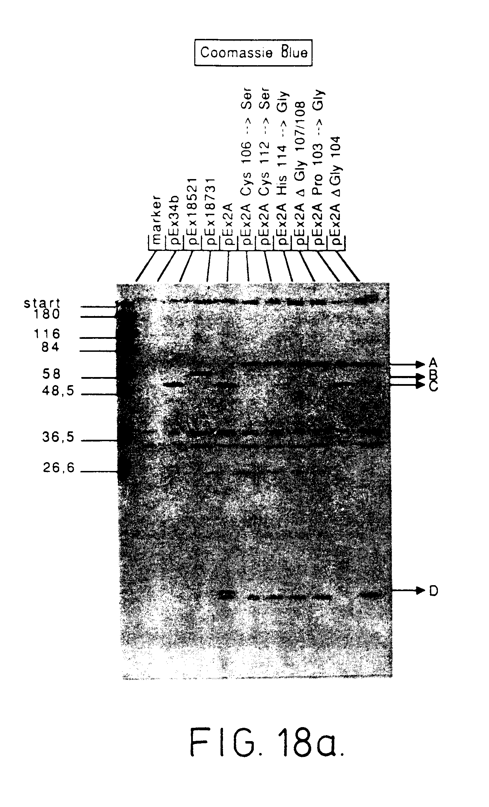

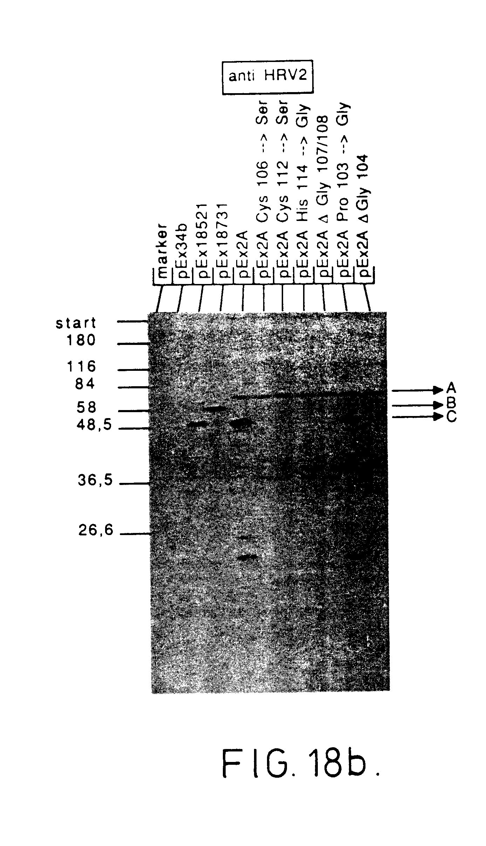

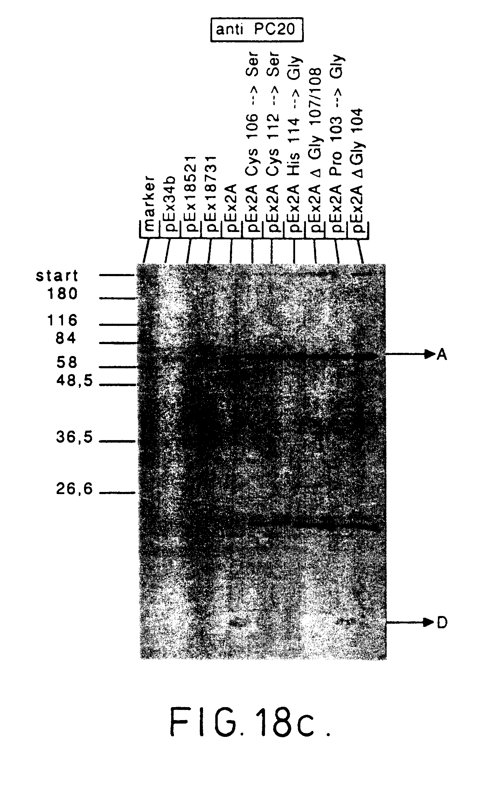

Durch Vergleich der Aminosäuresequenz der Proteasen 2A und 3C von Rhino-, Polio- und Coxsackieviren und durch in vitro Mutagenesestudien an Protease 3C von Polio (Ivanoff, L.A., et al. (1986) Proc. Natl. Acad. Sci. USA, 83, 5392 - 5396) kann angenommen werden, daß diese Enzyme ein Cystein und ein Histidin im aktiven Zentrum zur Proteolyse verwenden. Im Fall der Protease 2A von HRV2 kann man das wahrscheinlich aktive Zentrum den Aminosäuren 100 bis 140 zuordnen (siehe Fig. 15). Um die Rolle einiger hochkonservierter Aminosäuren in dieser Region zu untersuchen, wurde unter Verwendung einer Oligonukleotidkassette die in vitro Mutagenese und die Deletion einzelner Aminosäuren in dieser Region durchgeführt. Parallel dazu wurde diese Methode verwendet, um native Protease 2A zu exprimieren. Ausgehend von pEx18521 wurde durch Verdau mit Apa I (Nukleotidnummer 3458 der HRV2 cDNA) und Hind III (Restriktionsschnittstelle stammt aus der Polylinkerregion des Vektors; siehe Beispiel 1) nach Vorschrift des Herstellers (Biolabs) ein 264 bp langes DNA-Fragment gewonnen, welches durch zwei doppelsträngige Oligonukleotide mit Apa I/Hind III "sticky ends" ersetzt wurde. Dieses aus den zwei doppelsträngigen Oligonukleotiden WT 12 und WT 34 aufgebaute DNA-Fragment enthält, zum Unterschied zu dem 264 bp Fragment von pEx18521, nur mehr die 2A spezifische Sequenz. Zwei in Phase befindliche Stopkodons wurden zusätzlich hinter dem Kodon für die letzte Aminosäure (Glutamin) der 2A eingeführt. Dadurch bricht die Translation exakt nach der letzten Aminosäure von 2A (Glutamin 142) ab und es ergibt sich nach erfolgter Selbstspaltung zwischen VP1 und 2A die Möglichkeit eines Produktionssystems für eine native Protease 2A (siehe Fig. 17).
Das gereinigte, doppelsträngige ApaI/HindIII Fragment wurde sequenziert, ein positiver Klon ausgewählt und zu Expressionsstudien sowie zu Transaktivitätstests herangezogen. Die Induktion der Expression erfolgte wie in Beispiel 2 beschrieben und erbrachte das in Fig. 18 gezeigte Ergebnis. Bei Anfärbung mit Coomassie Blue (Fig. 18 Coomassie Blue) erkennt man bei Verwendung des Expressionssystems pEx2A das 50K Spaltprodukt, bestehend aus 98 Aminosäuren der MS2 Polymerase, dem C-terminalen Teil von VP3 und dem gesamten VP1. Bande D in Fig. 18 Coomassie Blue, Spur 5 stellt die native Protease 2A dar; die Bande bei ca. 65K (Bande A) repräsentiert die unprozessierte Form von pEx2A. Der Western blot mit HRV2 Antiserum wurde, wie in Beispiel 2 beschrieben, durchgeführt und zeigt ein ähnliches Muster wie bei Verwendung von pEx18521 (Fig. 18 Anti HRV2). Setzt man jedoch ein Antiserum (PC20; siehe Beispiel 7) ein, das gegen ein Peptid, welches die letzten 20 Aminosäuren von der Protease 2A von HRV2 enthält, gerichtet ist, so wird im Falle von pEx2A eine spezifische Bande erkannt, die der reifen 2A entspricht (Fig. 18 Anti PC20, Spur 5, Bande D). Um nun zu verifizieren, daß es sich dabei tatsächlich um die Protease 2A von HRV2 handelt, und um zu zeigen, daß die in vivo Spaltstelle (Kowalski et al., loc. cit.) erkannt worden war, wurde die Bande D aus dem Gel eluiert (wie in Beispiel 1 und 2 beschrieben) und N-terminal sequenziert (Hunkapiller, M.W. and Hood, L.E. (1983) Science, 219, 650 - 659). Die Sequenz war identisch mit den in Fig. 15 gezeigten Aminosäuren am N-Terminus von 2A. Überraschenderweise wurde bei Verwendung von Anti-PC20 Antiserum das Spaltprodukt von pEx18521 (2A+N-terminale Teile von 2B) von diesem Antiserum nicht erkannt. Möglicherweise bewirkt der Fusionsanteil eine andere Konformation des Proteins, die nicht vom Peptidantikörper erkannt werden kann oder der PC20 Antikörper reagiert nur mit dem freien C-Terminus von 2A.

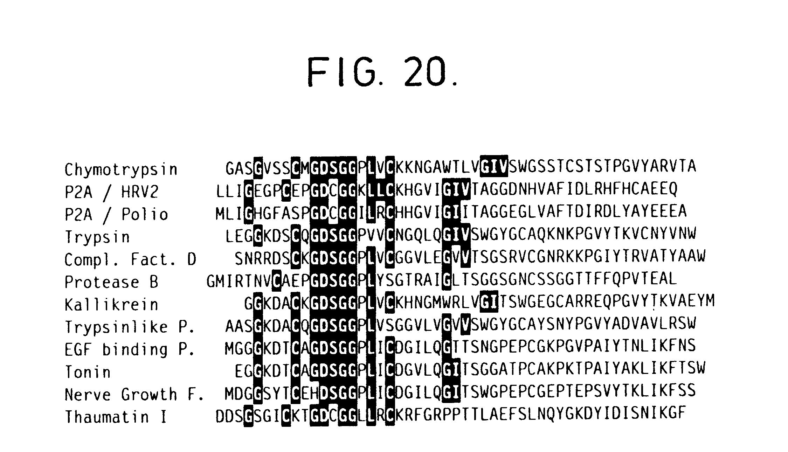

Unter Ausnützung der oben beschriebenen Oligonukleotidkassette wurden durch Austausch mit anderen Oligonukleotiden Mutationen bzw. Deletionen in das wahrscheinlich aktive Zentrum von 2A eingeführt siehe Figur 15 und 17). Diese Mutanten wurden, wie für pEx2A beschrieben, kloniert, sequenziert und exprimiert. Ebenfalls in Fig. 18 sind Coomassie Blue und Western blots mit HRV2- und PC20-Antiserum dieser Expressionsprodukte zu sehen. Alle Mutanten, bis auf pEx2A[Pro103-->Gly], zeigten keine proteolytische Aktivität mehr. Die Mutation Pro103-->Gly führt zu einer Reduzierung der proteolytischen Aktivität auf etwa 70% (siehe Fig. 18 Coomassie Blue und 18 Anti HRV2).
Obwohl offensichtlich ein Cystein als Nukleophil fungiert, ist überraschenderweise die Homologie der Region um das wahrscheinlich aktive Zentrum zu den Serinproteasen (wie z.B. Chymotrypsin) größer, als zu den Cysteinproteinasen (siehe Fig. 20). Die Vergleiche wurden mit den Proteinsequenz-Datenbanken PIR (Sidman, K.S., George, D.C., Barker, W.C. and Hunt, L.T. (1988) Nucleic Acids Res., 16, 1869 - 1871) und SWISSPROT (Cameron, G.N. (1988) Nucleic Acids Res., 16, 1865 - 1867) unter Anwendung des FASTP-Programms (Lipman, D.J. and Pearson, W.R. (1985) Science, 227, 1435 - 1451) erhalten. Bei Vergleich des wahrscheinlich aktiven Zentrums der Protease 2A (siehe Fig. 15) wurden überraschenderweise nur Homologien mit Serinproteasen, nicht aber mit Cysteinproteasen festgestellt. Ein ähnliches Ergebnis wurde 1986 von Gorbalenya et al. (Gorbalenya, A.E., et al. (1986) FEBS, 194, 253 - 257) für die Protease 3C von Poliovirus festgestellt, wobei allerdings eine wesentlich geringere Homologie gefunden wurde. Bemerkenswert ist die Tatsache, daß das Cystein 6 Aminosäuren nach dem wahrscheinlich aktiven Nukleophil, in den Serinproteasen und allen 2A Proteasen, nicht aber in den 3C Proteasen konserviert ist.
Bei der Protease 2A scheint es so, als fungiere ein Cystein als Nukleophil in der konservierten Umgebung (GDSGG) der Serinproteasen (Fig. 20). Mechanistisch gesehen gehören die 2A Proteasen demnach überraschenderweise der Klasse der Cysteinproteasen an, wobei allerdings eine für Serinproteasen typische Umgebung existiert. Bestimmte andere Reste sind ebenfalls stark konserviert, z. B. das Glycin und das Cystein vor dem aktiven Zentrum und das Glycin vor einer hydrophoben Aminosäure nach dem wahrscheinlich katalytischen Zentrum (siehe Fig. 15). Da die Homologie mit Cysteinproteasen, wie oben beschrieben, sehr gering ist, ist es ebenfalls zweifelhaft, ob das konservierte Histidin (His114 in HRV2 2A) als funktionelles Analogon zum Histidin der Cysteinproteasen angesehen werden kann (z.B. Papain). Die Mutation des Histidin 114 in HRV2 zeigte zwar, daß es für die Aktivität essentiell ist, nicht aber ob es direkt am katalytischen Mechanismus beteiligt ist. Außerdem findet man eine konservierte Asparaginsäure und ein zweites konserviertes Histidin in allen bis jetzt untersuchten 2A und 3C Proteasen. Dies könnte ein Hinweis auf die mögliche Existenz eines "charge relay system", ähnlich dem im Chymotrypsin, sein. Die Tatsache, daß es bei Austausch der beiden Cysteine von pEx2A(106 und 112) in beiden Fällen zu einem Verschwinden der proteolytischen Aktivität kommt, steht im scheinbaren Widerspruch zu den Ergebnissen in Beispiel 4. Man muß jedoch bedenken, daß es sich bei der Mutation in pEx18521 um ein Expressionsprodukt handelt, welches zum Unterschied zu pEx2A einen zusätzlichen C-terminalen Fusionsproteinanteil von 39 Aminosäuren von 2B plus 22 Aminosäuren (aus dem Vektor) aufweist, der durchaus einen großen Einfluß auf die Stabilität und damit auf die Aktivität von 2A ausüben kann (z. B. Knüpfung einer anderen Disulfidbrückenbindung).

Außerdem wurden wie in Beispiel 8 beschrieben, Cysteine gegen Serine (etwa gleich großer Raumbedarf) ausgetauscht, während in Beispiel 4 Cysteine durch Tryptophan-Reste ersetzt wurden (wesentlich größerer Raumbedarf), was durchaus zu strukturellen Veränderungen führen kann.

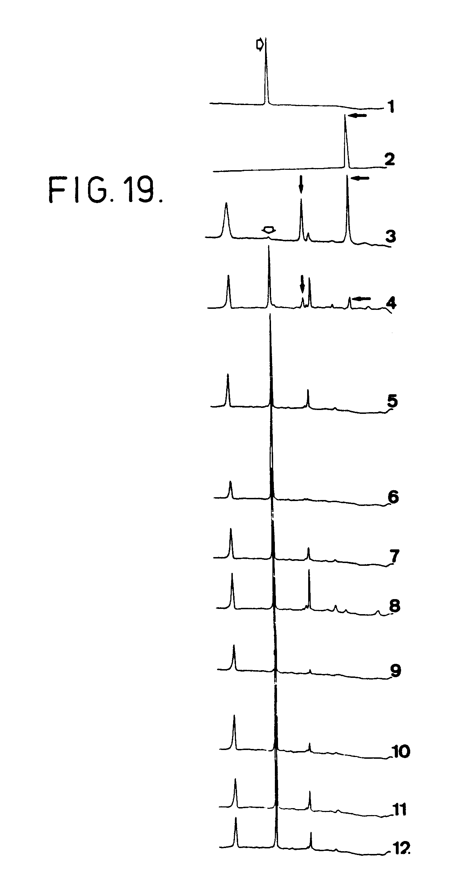

Zur Überprüfung der in vitro Transaktivität ("trans-assay") wurde als Peptid-Substrat ein 16 Aminosäuren langes Peptid eingesetzt (Ac-TRPIITTAGPSDMYVH). Es enthält 8 Aminosäuren vor und 8 Aminosäuren nach der erwarteten Spaltstelle von 2A (Spaltung findet zwischen den unterstrichenen Aminosäuren statt). Das ursprüngliche 16 Aminosäuren lange Peptid-Substrat und ein Referenzpeptid, welches das C-terminale Spaltprodukt (GPSDMYVH) repräsentiert, wurden auf einer HPLC Säule aufgetrennt. Die Expression der 2A-Expressionsysteme wurde wie in Beispiel 2 beschrieben im E.coli-Stamm 537 induziert. Nach Entfernung unlöslichen Materials wurde der Überstand mit einer wäßrigen Peptidlösung von Ac-TRPIITTAGPSDMYVH versetzt und inkubiert. Nach Aufarbeitung wurde der Peptid-enthaltende Überstand chromatographisch aufgetrennt. Figur 19 zeigt die HPLC Profile der Peptide nach Inkubation des Peptid-Substrates in verschiedenen bakteriellen Extrakten. Bei Verwendung des Extraktes von E. coli Zellen, die das pEx2A Expressionssystem besitzen, verschwindet der Peak t_{R}=20,8 min (ungespaltenes Peptid-Substrat; Ac-TRPIITTAGPSDMYVH; offener, dicker Pfeil; absorbiert bei 214 nm und bei 280 nm). Dafür sind zwei neue Peaks erkennbar; der eine repräsentiert das N-terminale Spaltprodukt (Ac-TRPIITTA; voller, vertikaler Pfeil; t_{R}=16,8 min; absorbiert nur bei 214 nm); der zweite Peak repräsentiert das C-terminale Spaltprodukt (GPSDMYVH; voller horizontaler Pfeil; t_{R}=12,2 min; absorbiert bei 214 nm und bei 280 nm). Die Tatsache, daß der Peak mit der Retentionszeit t_{R}=12,2 min tatsächlich das C-terminale Spaltprodukt darstellt, wurde einerseits durch Komigration dieses Spaltpeptides mit dem Referenzpeptid (siehe Fig. 19, Nummer 2), andererseits wie oben beschrieben durch N-terminale Sequenzierung verifiziert. Das Spaltprodukt mit der Retentionszeit t_{R}=16,8 min kann aufgrund seines blockierten N-Terminus nicht sequenziert werden. Neben pEx2A zeigt nur noch das Expressionsystem pEx2A[Pro103-->Gly] eine, wenn auch drastisch reduzierte Transaktivität (etwa 10%; siehe Fig. 19, Nummer 4). Alle anderen Mutanten von pEx2A zeigen keine Transaktivität (Fig. 19). Besonders überraschend und bemerkenswert ist die Tatsache, daß auch das proteolytisch aktive Expressionssystem pEx18521 keine Transaktivität aufweist. Offensichtlich beeinflußt der C-terminale Fusionsanteil (39 Aminosäuren von 2B und 22 Aminosäuren von der Vektor DNA) aufgrund der Löslichkeit des Fusionsproteins von 2A die Aktivität in trans, nicht aber die Aktivität in cis (Fig. 19, Nummer 5).

Durch die vorliegende Erfindung wird ein Expressionssystem bereitgestellt, mit dem es möglich ist, potentielle Inhibitoren gegen P2A aufzufinden und zu optimieren; eine Grundvoraussetzung für die Umsetzung des überzeugend erscheinenden therapeutischen Konzepts, durch Unterdrückung der proteolytischen Aktivität der viralen Protease P2A den für eine virale Infektion erforderlichen Reifungsprozeß des viralen Systems zu unterbinden. Durch die ausgeprägte Homologie der P2A-Region auch zu anderen Gruppen der Picornaviridiae ist es auch denkbar, die durch dieses erfindungsgemäße System aufgefundenen Inhibitoren therapeutisch auch gegen Infektionen anderer Picornaviren einzusetzen.

Die vorliegende Erfindung stellt erstmals einen wirkungsvollen "trans assay" für die Protease 2A bereit. Es konnte gezeigt werden, daß Peptide als Spaltstellen-Analoga verwendet werden können, wodurch eine detaillierte und rasche biochemische Charakterisierung der Protease 2A ermöglicht wird. Mit Hilfe dieses erfindungsgemäßen "trans assay" ist es damit möglich, Substanzen auf ihre Inhibitorwirkung bezüglich der Protease 2A-Aktivität zu überprüfen; er gewährleistet also ein Inhibitorscreening auf breiter Basis. Mit seiner Hilfe ist es möglich, einen spezifischen viralen Protease-2A-Inhibitor zu finden. Gegenstand der vorliegenden Erfindung ist auch die Verwendung des Expressionsystems als Testsystem für Inhibitoren viraler Proteasen, bevorzugt für P2A-Inhibitoren, besonders bevorzugt für HRV2-P2A-Inhibitoren.

Die vorliegende Erfindung umfaßt daher im einzelnen ein:
- Expressionssystem aus einem Plasmidanteil und einem Insert, bei dem der Plasmidanteil in der Lage ist, ein von dem Insert kodiertes Fusionsprotein effektiv zu exprimieren und bei dem das Insert für ein Fusionsprotein aus einem enzymatisch aktiven Anteil einer rhinoviralen 2A Protease und einem von diesem autokatalytisch abspaltbaren Polypeptidanteil kodiert.
- Expressionssystem, bei dem der Plasmidanteil eine prokaryontische ribosomale Bindungsstelle, eine kodierende Region für einen die Stabilität des exprimierten, von dem Insert kodierten Fusionsproteins in der Zelle erhöhenden Fusionsanteils, einen das Fusionsprotein kontrollierenden Promotor, eine Polylinkerregion in drei verschiedenen Leserahmen, vorzugsweise mit Schnittstellen für EcoRI, BamHI, HindIII, PstI, BglII und XbaI und die "ori"- und Ampicillin-resistenz-region von pBR322 enthält.
- Expressionssystem, bei dem das Insert ein wie oben angegebenes DNA-Molekül im richtigen Leserahmen ist.
- Verwendung der Expressionsysteme als Testsysteme für Inhibitoren viraler Proteasen.
- Verwendung der Expressionsysteme als Testsysteme für P2A-Inhibitoren, bevorzugt für HRV2-P2A-Inhibitoren.
- "Trans-assay" als Testsystem für virale Inhibitoren, das eines der oben angegebenen Expressionssysteme enthält.
- "Trans-assay" als Testsystem für virale Inhibitoren, bei dem das Expressionssystem ein wie oben angegebenes DNA-Molekül im richtigen Leserahmen enthält.

### LEGENDEN ZU DEN FIGUREN

Fig. 1
   Das picornavirale Polyprotein und seine proteolytischen Spaltstellen.
Fig. 2
   Spaltstellen im Polyprotein von HRV2, HRV89, HRV14 und Poliovirus Typ 1; diejenigen Aminosäuren von HRV2 und 89, die durch Proteinsequenzierung ermittelt wurden, sind unterstrichen.
Fig. 3
   Konstruktion von pEx34c x 18521
Fig. 4
   Aufbau des Expressionsplasmides pEx34c x 18521
Fig. 5
   Elektrophoretische Trennung der Expressionsprodukte von pEx34c x 18521 (Spur 1) und PEx34c x 18731 (Spur 2), sowie der viralen Hüllenproteine von HRV2 (Spur HRV2) auf einem 10%igen SDS-Polyacrylamidgel und Anfärbung mit Coomassie-Brillant-Blau.
Fig. 6
   Western blot der Expressionsprodukte von pEx34c x 18521 bzw. 18731 mit einem polyklonalen anti-VP1 Serum; in Spur 1 bis 3 ist das Expressionsprodukt von 3 verschiedenen Klonen von 18521, in Spur 4 von 18731 aufgetrennt. Die nur schwach erkennbaren höhermolekularen Banden in Spur 1 bis 3 repräsentieren das noch unprozessierte Expressionsprodukt von 18521.
Fig. 7
   Autoradiogramm der "in vitro" Transkription und Translation von pEx34c x 18521 und pEx34c x 18731
   - Spur 1: pAT 153 (Kontrollplasmid; β-Lactamase)
   - Spur 2 und 3: p18731 (Deletionsmutante von 18521)
   - Spur 4 und 5: p18521 (Expressionsprodukt mit aktiver Protease P2A von HRV2)

   - a: unprozessiertes Fusionsprotein von 18521 bestehend aus: MS2-Pol. -VP1- P2A und einem N-terminalen Teil von P2B
   - b: durch Deletion verkürztes, nicht mehr prozessierbares Fusionsprotein von p18731
   - c: prozessiertes Fusionsprotein von p18521 bestehend aus: Ms2-Pol. und VP1 (Spaltprodukt ist nicht nachweisbar in p18731)
   - d: β-Lactamase
   - e: wahrscheinlich virale Protease P2A und ein N-terminaler Teil von P2B; entstanden durch autokatalytische Spaltung von p18521 (ebenfalls nicht vorhanden in p18731)
Fig. 8
   Aminosäuresequenzvergleich der wahrscheinlichen katalytischen Region der viralen Proteasen P2A und P3C von HRV2, 14 und 89, sowie von Poliovirus Typ 1. Die unterstrichenen Aminosäuren (Cystein und Histidin; mit dicken Pfeilen gekennzeichnet) sind wahrscheinlich am Aufbau des katalytischen Zentrums beteiligt.
Fig. 9
   Nukleotid- und Aminosäuresequenz der viralen Protease P2A von HRV2. Die dicken Pfeile geben die Aminosäureposition der beiden Cysteine (106 und 112) und des Histidins (in Position 114), welche möglicherweise am Aufbau des katalytischen Zentrums beteiligt sind, an.
Fig. 10
   Abschnitt der cDNA des katalytischen Zentrums von HRV2-P2A sowie die Sequenz der Oligonukleotide zur Mutagenese der beiden Cysteinreste in Position 106 und 112
Fig. 11
   Autoradiogramm der in vitro Transkription/Translation des mutierten pEx34c x 18521:
   - Spur 1:: Austausch des Cysteins in Position 112 gegen Phenylalanin in pEx34c x 18521
   - Spur 2:: nicht mutiertes pEx34c x 18521
   - Spur 3:: Austausch des Cysteins in Position 106 gegen Phenylalanin in pEx34c x 18521
   - Spur 4:: pAT153
Fig. 12
   In vivo Markierung von P2A
   - 1: 18521; Plasmid mit aktiver Form von P2A, bestehend aus: Teil der MS2-Polymerase/VP1/P2A/N-Terminus von P2B (siehe Beispiel 1)
   - 2: 13L; Deletionsmutante von 18521;
   HRV2-kodierende Region endet ca. 18 Aminosäuren vor dem C-Terminus von P2A
   - P: Pellet
   - S: Überstand
   Die Angaben 37°C und 42°C beziehen sich auf die im Beispiel 5 verwendeten Expressionstemperaturen.
   - a: unprozessiertes Expressionsprodukt von 18521
   - b: nicht prozessierbares Expressionsprodukt von 13L
   - c: prozessiertes Expressionsprodukt von 18521 bestehend aus: Teil der MS2-Polymerase/VP1
   - d: wahrscheinlich P2A repräsentierendes Peptid bestehend aus: P2A/N-Terminus von P2B; nicht anwesend in der nicht prozessierenden Deletionsmutante 13L
Fig. 13
   Konstruktionsschema zur Herstellung von Deletionsmutanten der C-terminalen HRV2-P2A-Region
Fig. 14
   Auftrennung der am C-Terminus deletierten Expressionsprodukte von pEx34c x 18521 auf einem 10%igen SDS-Polyacrylamidgel und Anfärbung mit Coomassie-Brillant Blau (obere Bildhälfte), sowie ein Western blot mit einem polyklonalen Serum gegen VP1 von HRV2 (untere Bildhälfte).
   Die Spuren HRV2 (Positivkontrolle für VP1). pEx34c (Negativkontrolle) und 18521 (Positivkontrolle für proteolytisches Processing durch P2A) dienen als Referenzmarker. Außer 13W zeigen alle anderen Deletionsmutanten kein proteolytisches Processing mehr.
Fig. 15
   Aminosäuresequenz der HRV2-Protease-2A-Region (fette Buchstaben). Die Großbuchstaben weisen auf die zwischen Rhino-, Polio- und Coxsackieviren identischen Aminosäuren hin. Doppelpfeile zeigen die Position und die Art des Austausches bzw. der Deletion an, die zur Charakterisierung des wahrscheinlich aktiven Zentrums und des C-Terminus der Protease 2A von HRV2 herangezogen wurden.
Fig. 16
   Proteinmuster (Coomassie Blue) von pEx18521 (Spur 1) und der Mutante pEx18521[Arg134-->Gln].
Fig. 17
   Oligonukleotidkassette zur Mutagenese des wahrscheinlich aktiven Zentrums der Protease 2A von HRV2. Durch Kombination der beiden doppelsträngigen Oligonukleotide WT12+WT34 erhält man die kodierende Region für die Wildtyp-Protease 2A. Nach der letzten Aminosäure von 2A (Glutamin 142) wurden zwei Stoppkodons eingefügt. Kombiniert man das doppelsträngige Oligonukleotid WT34 anstelle von WT12 mit einem Oligonukleotid, das aufgrund seiner geänderten Basensequenz eine geeignete Mutation oder Deletion besitzt, kann man jede gewünschte Veränderung in der Aminosäuresequenz in dieser Region herbeiführen (hier gezeigt am Beispiel für die Konstruktion der Wildtyp-2A und den Mutationen für Cys106-->Ser, Cys112-->Ser und His114-->Gly).
Fig. 18 a-c
   Zeigt das Proteinmuster von pEx34c (Negativkontrolle), pEx18521, pEx18731, pEx2A und sämtlichen Mutanten von pEx2A im wahrscheinlich aktiven Zentrum von 2A.
   - Bande A: entspricht dem unprozessierten Expressionsprodukt (65 K) von pEx2A bzw. von den Mutanten von pEx2A
   - Bande B: entspricht dem Expressionsprodukt (58 K) von pEx18731
   - Bande C: entspricht dem prozessierten Expressionsprodukt (50 K; MS2-Polymeraseanteil+C-terminaler Teil von VP3+gesamtes VP1) von pEx18521, pEx2A und pEx2A[Pro103-->Gly]
   - Bande D: entspricht der reifen Protease 2A (15 K)
Fig. 18a Coomassie Blue;
   mit Coomassie Blue gefärbtes Proteingel
Fig. 18b Anti HRV2;
   Western blot des gleichen Gels mit einem polyklonalen Antiserum gegen HRV2
Fig. 18c Anti PC20;
   Western blot des gleichen Gels mit einem polyklonalen Antiserum gegen PC20 (Peptid das aus den letzten 20 Aminosäuren von 2A aufgebaut ist; siehe Beispiel 7)
Fig. 19
   HPLC Profile von Peptiden, welche nach Inkubation (siehe Beispiel 9) aus Überständen von verschiedenen 2A Expressionssystemen isoliert wurden.
   - 1: reines Spaltpeptid (Ac-TRPIITTAGPSDMYVH), t_{R}=20,8 min (offener großer Pfeil)
   - 2: reines C-terminales Spaltprodukt (GPSDMYVH), t_{R}=12,2 min (fetter horizontaler Pfeil)
   - 3: Peptidmuster bei Verwendung des pEx2A Überstandes, vertikaler fetter Pfeil zeigt das zweite Spaltprodukt an (Ac-TRPIITTA),
   t_{R}=16,8 min
   - 4: pEx2A[Pro103-->Gly]
   - 5: pEx18521
   - 6: pEx18731
   - 7: pEx2A[Cys106-->Ser]
   - 8: pEx2A[His114-->Gly]
   - 9: pEx2A[Cys112-->Ser]
   - 10: pEx2A[ΔGly104]
   - 11: pEx2A[ΔGly107/108]
   - 12: pEx34c
Fig. 20
   Vergleich der Aminosäuresequenz des wahrscheinlich aktiven Zentrums der Protease 2A von HRV2 (von Aminosäure 94 bis 142) mit den Proteinsequenzdatenbanken PIR und SWISSPROT.

Die nachfolgenden Beispiele sollen die Erfindung nur erläutern, sie jedoch in keiner Weise irgenwie einschränken.

### Beispiel 1:

### Herstellung eines aktiven und inaktiven P2A-Enzymsubstrates von HRV2 zur Expression in E. coli

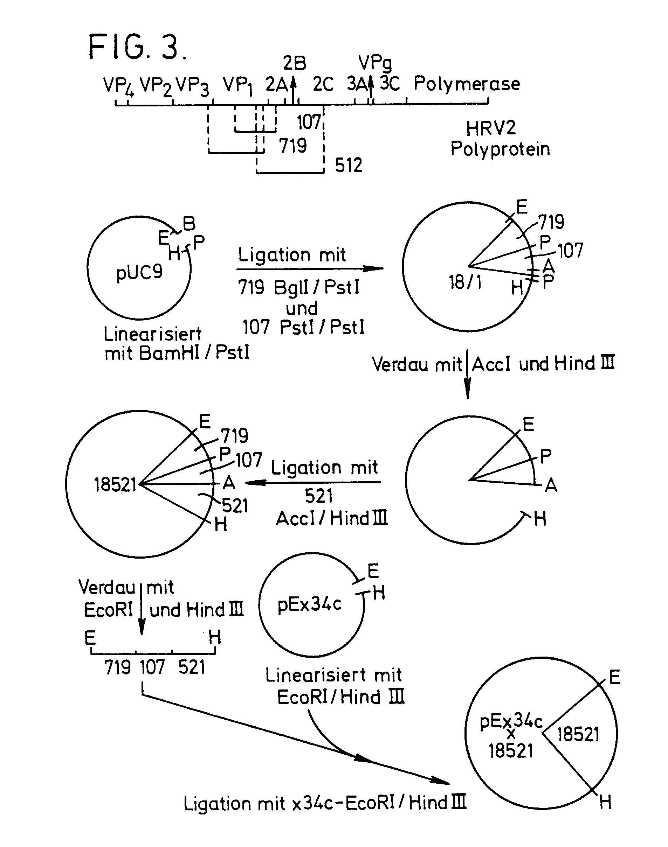

Ausgehend von pUC9 und den cDNA-Klonen von HRV2 (Fig. 3) wurde ein Expressionssystem für P2A konstruiert. Zunächst wurden ca. 10 µg pUC9 durch Doppelverdau mit BamHI und PstI in der Polylinkerregion geöffnet. Die linearisierte Form von pUC9 wurde von Spuren des ungeschnittenen Plasmids mit Hilfe von Whatman DE81-Papier getrennt (Dretzen, G.M., Bellard, P., Sassone-Corsi und Chambon, P.; 1981, Anal. Biochem. 112, 295 - 298). Dazu wurde nach Auftrennung der DNA-Fragmente am Agarosegel vor und hinter die zu isolierende DNA-Bande ein Schlitz geschnitten, in den jeweils ein Streifen DE81-Papier gesteckt wurde. Die Elektrophorese wurde weitergeführt (Gel soll nicht mit Puffer bedeckt sein), bis das gewünschte DNA-Fragment vollständig an den vorderen DE81-Streifen gebunden worden war. Der hintere DE81-Streifen verhinderte eine Verunreinigung durch größere DNA-Fragmente. Das DE81-Papier mit dem gebundenen DNA-Fragment wurde in ein 1,5 ml Eppendorfröhrchen (mit einem Ausflußloch im Gefäßboden und darübergelagerter Polyallomerwolle) transferiert und zweimal 5 min mit je 400 µl Waschpuffer (0,2M NaCl, 25mM TrisHcl pH=8,0, 1mM EDTA) gewaschen, wobei durch kurze Zentrifugation (ca. 1 sek) die Waschlösung im zweiten darunter befindlichen Eppendorfgefäß aufgefangen wurde. Anschließend wurde die gebundene DNA zweimal durch 15 Minuten dauernde Inkubationen in je 200 µl Elutionspuffer (1M NaCl, 25mM TrisHcl pH=7,5, 1mM EDTA) vom DE81-Papier gewaschen. Die 400 µl Eluat wurden 10 min in der Eppendorfzentrifuge (15000g) zentrifugiert, um Papierstückchen zu entfernen. Der Überstand wurde vorsichtig in ein neues Eppendorfgefäß transferiert, mit 800 µl 96% Ethanol versetzt, bei -20°C ausgefällt (ca. 2 Stunden), zweimal mit 70% Ethanol gewaschen und getrocknet. Parallel dazu wurde das Plasmid von Klon 719 mit BglII und PstI und das Plasmid von Klon 107 mit PstI verdaut (Fig. 3). Diese beiden Plasmide sind pBR322-Vektoren und enthalten HRV2-cDNA-Fragmente, welche über homopolymere G-C-Regionen in pBR322 insertiert worden waren. Das BglII/PstI-Fragment von Klon 719 repräsentiert die HRV2-cDNA Region von 2145 - 2421 (siehe Fig. 3); das PstI/PstI-Fragment von Klon 107 deckt die anschließende Region 2421-3100 ab. Diese beiden Fragmente wurden in die BamHI und PstI Stelle von pUC9 insertiert, wobei beide Schnittstellen (BglII und BamHI) zerstört wurden. Diese Konstruktion wurde p18 genannt (siehe Fig. 3).
Um für die Transformation kompetente Zellen zu erhalten, wurde eine modifizierte Vorschrift von Mandel und Higa (Mandel, M. und Higa, A., 1970, J. Mol. Biol. 53, 159-162) angewandt. Dazu wurden 0,5 ml einer "über Nachtkultur" von E. coli Stamm HB 101 in 50 ml LB-Medium (10 g/l Trypton, 5 g/l Hefeextrakt, 10 g/l NaCl) überimpft, bis zu einer OD600 von ca. 0,4 hochgezüchtet und anschließend 5 min bei 5 k und 4°C pelletiert. Die Bakterien wurden dann in 25 ml 0,1M MgCl₂ (eiskalt) vorsichtig resuspendiert, 5 min auf Eis gestellt und abermals 5 min bei 5 k und 4°C zentrifugiert. Das Pellet wurde in 25 ml 0,1M CaCl₂ (eiskalt) resuspendiert, 4 Stunden auf Eis gestellt und 5 min bei 5 k und 4°C zentrifugiert. Die Zellen wurden in 2,5 ml 1x Lagerpuffer (0,1M CaCl₂/Glycerin = 4/l% v/v) aufgenommen, 20 min auf Eis gestellt, in 100 µl Portionen aliquotisiert und in flüssigem Stickstoff schockgefroren, sowie bei -80°C gelagert. Zu 100 µl auf Eis aufgetauter kompetenter Zellsuspension wurden 5 µl des oben beschriebenen Ligationsansatzes zugegeben, die Zellen 1 Stunde auf Eis und 2 min bei 42°C inkubiert und abschließend 5 min auf Eis gestellt. Vor dem Ausplattieren der Zellen wurden 900 µl LB-Medium zugesetzt, 10 min bei 37°C inkubiert und je 200 µl Zellsuspension auf LB-Agar-Platten (1,5%iger Agar in LB-Medium mit 100 mg/l Ampicillin) aufgebracht und über Nacht inkubiert. Mit dem Plasmid p18 wurden wie oben angeführt kompetente JM 101 Zellen transformiert. Einige der erhaltenen Amp^{r} Klone wurden einer Restriktionsanalyse unterworfen und von einem der positiven Klone (18/1) wurde Plasmid-DNA im Großmaßstab gewonnen (T. Maniatis et al. 1982, Molecular Cloning: A Laboratory Manual; Cold Spring Harbor, 86ff). Das Plasmid wurde anschließend über eine Sephacryl-S-1000 Säule gereinigt (Durchmesser 0,9cm, Länge 20cm). Als Elutionspuffer wurde ein TE-Puffer verwendet. Das Eluat wurde in ca. 0,5 ml Fraktionen aufgetrennt und die einzelnen Fraktionen bei 260 nm ausgemessen (üblicherweise erscheint der Plasmidpeak zwischen der 9. und 14. Fraktion). Der Beginn des RNA-Peaks ist ab der 17. Fraktion zu erwarten (OD über 3,0). Die in Frage kommenden Fraktionen wurden vereinigt, lyophilisiert, das Plasmid in 0,5 ml TE-Puffer aufgenommen, 5 min bei 65°C inkubiert, mit Phenol/Chloroform und Chloroform extrahiert, ausgefällt, zentrifugiert, getrocknet und in 100 µl TE-Puffer gelöst. Etwa 10 µg von Plasmid 18/1 wurden mit AccI und HindIII (die AccI Stelle stammt von der HRV2-cDNA, die HindIII Stelle von der Polylinkeregion von pUC9) geschnitten. Parallel dazu wurde der Klon 521 mit AccI und HindIII verdaut. Das AccI/HindIII-Fragment von Klon 521 umfaßt die HRV2-cDNA von Nukleotidnummer 3075-3698 (Fig. 3). Das AccI/HindIII-Fragment von 521 wurde in 18/1 (AccI/HindIII) ligiert und damit kompetente JM 101, wie oben beschrieben transformiert. Die erhaltenen Kolonien wurden durch Restriktionsanalyse überprüft (EcoRI, PstI, AccI und HindIII) und die Plasmid DNA einiger Klone wurde sequenziert. Ein Klon, der die HRV2-Sequenz von 2145 - 3698 und den richtigen Leserahmen aufwies, wurde 18521 benannt und zur Expression ausgewählt. In der Konstruktion 18521 sind die homopolymeren G-C-Regionen, die noch von der Klonierung in pBR322 herstammen 5′ seitig von der HindIII Stelle vorhanden. p18521 wurde mit EcoRI und HindIII geschnitten, das Fragment mit DE81 Papier isoliert und in pEx34c (EcoRI/HindIII) durch Ligation insertiert. pEx34 ist ein 3,0 kb Expressionsvektor (ein Derivat von pPLc24; E. Remault, P. Stanssens und W. Fiers; 1981, Gene 15, 81-93) welcher folgende Abschnitte enthält:
- -: die prokaryontische ribosomale Bindungsstelle
- -: einen Teil der kodierenden Region für die ersten 98 N-terminalen Aminosäuren der MS2-Polymerase; dieser Fusionsanteil weist hydrophobe und basische Aminosäuren auf und bewirkt die Herabsetzung der Löslichkeit des Fusionsproteins und erhöht die Stabilität des exprimierten Produktes in der Zelle.
- -: das Fusionsprotein steht unter der Kontrolle des linken lambda-Promotors
- -: eine kleine Polylinkerregion in 3 verschiedenen Leserahmen (pExa,b und c) mit Schnittstellen für EcoRI, BamHI und HindIII ermöglicht das Einsetzen von geeigneten DNA-Fragmenten in Phase hinter dem Fusionsproteinanteil
- -: die "ori"- und die Ampicillin-resistenz-region von pBR322.

E. coli W6(lambda) exprimiert konstitutiv das Gen für den Wildtyp lambda-Repressor und eignet sich zum Hochzüchten der pEx-Plasmide. E. coli 537 dagegen trägt die cI 853-lambda- Repressor-Mutation (inaktiv bei 42°C) auf einem weiteren Plasmid, das auch ein Kanamycin Resistenzgen trägt (K. Strebel, E. Beck, K. Strohmaier und H. Schaller; 1986, J. Virol. 57, 983-991).
Durch Insertion des EcoRI/HindIII Fragmentes von p18521 in pEx34c konnte ein Expressionssystem erhalten werden, welches die Region: (VP3)-VP1-P2A-(P2B) von HRV2 (2145-3698; siehe Fig. 4) umfaßt.
Dieses Expressionssystem Produziert ein als Substrat dienendes virales Polypeptid, das gleichzeitig auch P2A-Protease-Aktivität aufweist.
Um nun ein inaktives Enzymsubstrat für P2A zu erhalten wurde der Expressionsvektor pEx34c x 18521 in E. coli W6(lambda) hochgezüchtet. Wie oben beschrieben wurde der Vektor nach der Großmaßstabpräparationstechnik aus 500 ml einer Übernachtkultur isoliert. 2 µg von pEx34c x 18521 wurden mit HindIII verdaut und mit Hilfe von DE81 wie oben beschrieben gereinigt. Anschließend wurde wie folgt der linearisierte Vektor mit Bal3l - Nuklease verdaut: ca. 1 µg des mit HindIII linearisierten Vektors wurden mit 1U Bal3l- Nuklease (Biolabs) in 20 mM TrisHCl pH=8, 600 mM NaCl, 12 mM MgCl2 und 1 mM EDTA inkubiert. Aliquote Proben wurden nach 1-2-3-4-5-6 und 8 Minuten entnommen und durch Zugabe von EDTA (Endkonzentration 30 mM) wurde der Verdau gestoppt. Die DNA wurde durch Ethanolpräzipitation wiedergewonnen und 100 ng des Plasmids wurden mit 100 U T4-Ligase in 10 mM TrisHCl pH=7,5, 6 mM MgCl2, 6 mM BME und 1 mM ATP über Nacht bei 15°C inkubiert. Der T4-DNA-Ligaseansatz wurde direkt zur Transformation von E. coli W6(lambda) verwendet. Einige der Klone wurden gepickt und die jeweilige Plasmid DNA wurde nach Maxam und Gilbert sequenziert (Maxam, A. und Gilbert, W., 1980, Nucleic Acids Res. 65, 499 - 560). Ein Klon dessen cDNA mit der HRV2-Nukleotidnummer 3321 (siehe Skern, T. et al., 1985, Nucleic Acids Res. 13, 2111 - 2126) endet, wurde 18731 benannt. Diese Deletionsmutante von 18521 wurde als inaktives P2A-Enzymsubstrat zu Expressionsstudien herangezogen.

### Beispiel 2:

### Expression und Nachweis der Fusionsproteine

Das Plasmid pEx34c x 18521 und Plasmide von Klonen des Bal3l-Verdaus von pEx34c x 18521 wurden in E. coli Zellen transferiert. Die Zellen wurden über Nacht bei 28°C in LB-Medium (+100 mg Ampicillin/l und 25 mg Kanamycin/l) hochgezüchtet. Die Kulturen wurden anschließend 1:5 mit vorgewärmten (42°C) LB-Medium ohne Antibiotika verdünnt (Induktion des lambda-PL-Promotors) und für 2 Stunden unter heftigem Schütteln bei 42°C inkubiert. Nach 2 Stunden wurden die Zellen von 1 ml der Kultur geerntet (2 min in der Eppendorfzentrifuge, 4°C) und in 500 µl kalten Sonikierungspuffer (150 mM NaCl, 50 mM TrisHCl pH=8 und 1 mM EDTA) resuspendiert. Das Aufbrechen der Zellen wurde mit Hilfe eines M. S. E. Ultrasonic Power Gerätes vorgenommen (3 mal je 5 sek; 1,7 Amp.), wobei zwischen den einzelnen Sonikierungen eine Pause von 45 sek eingeschaltet wurde, um ein Überhitzen der Proben zu vermeiden. Unlösliches Material wurde anschließend durch 2 min Zentrifugation in der Eppendorfzentrifuge gewonnen und die Pellets in 200 µl Sample buffer (4% SDS, 125 mM TrisHCl pH=6,8, 10% BME, 10% Glycerin und 0,02% Bromphenolblau) gelöst. Nach Erhitzen auf 95°C für 4 min, wurden je 10 µl der Proben auf einem 10% SDS-PAA-Gel (Lämmli, U. K., 1970, Nature 227, 680-685) aufgetrennt. Kontrollexperimente zeigten, daß alle exprimierten Proteine im Sonikierungspuffer unlöslich sind. Die Gele wurden anschließend mit Coomassie Brilliant Blue wie folgt gefärbt:
- -Färbung:: 30- 60 min in 50% Methanol, 10% Essigsäure und 0,1% Coomassie Brilliant Blue
- - Entfärbung:: über Nacht in 5% Methanol und 10% Essigsäure
- - Glycerinbad:: 30 min in 7% Essigsäure und 2% Glycerin
- - Ethanolbad:: 1 bis 2 min in 96% Ethanol
- - Trocknen:: auf 3MM Papier; 2-3 Stunden bei 80°C auf Geltrockner (Hoefer, SE 1160)

In Fig. 5 ist ein typisches Bild einer Coomassie Brilliant Blue Anfärbung einer Expression von pEx34c x 18521 in 537 zu sehen. Ebenfalls aufgetrennt wurde die Deletionsmutante 18731 (siehe Fig. 5). pEx34c x 18731 endet bei Nukleotidnummer 3321 und besitzt daher das wahrscheinlich aktive Zentrum von P2A nicht mehr (siehe auch Fig. 9).
Um die antigenische Spezifität dieser exprimierten Formen von 18521 und 18731 aufzuzeigen wurde ein Western blot mit Hilfe eines polyklonalen Serums gegen VP1 durchgeführt (VP1 ist integraler Bestandteil beider Expressionsplasmide pEx34c x 18521 bzw. 18731). Der Western blot wurde wie folgt durchgeführt:
Für den Elektrotransfer der aufgetrennten Proteine vom Gel auf die Nitrozellulose wurden 4 Lagen 3MM Papier (Whatman) und 1 Lage Nitrozellulose (Schleicher und Schuell, BA85, 0,45 um) genau den Abmessungen des Trenngels entsprechend zurechtgeschnitten und in Transferpuffer präinkubiert:
20 mM Tris-Base
150 mM Glycin
20% Methanol p.a.
(pH=8,8; braucht nicht mehr titriert zu werden).

Die Zusammenstellung des "Transfer-Sandwich" erfolgte nach dem unten angeführten Schema (Luftblasen vermeiden!):
- Pol --> scotch brite --> 2 Lagen 3 MM --> Gel --> Nitrozellulose --> 2 Lagen 3 MM --> scotch brite --> + Pol
Das Proteingel wurde ebenfalls vor dem Zusammenbau 2 min in Transferpuffer äquilibriert. Der Transferpuffer kann auch als 10 x Lösung (24,2g Tris und 112,6 g Glycin pro Liter ohne Methanol) hergestellt werden. Der Transfer wurde im Transferpuffer bei ca. 1 Ampere, 2 Stunden in einer Protein-Blot-Apparatur in Anwesenheit von 0,1% Empigen BB (Alkyldimethylammoniumbetain; Nr. 62 852, Marchon France S.A.) durchgeführt (R.E. Mandrell et al.; J. Immunol. Meth. 67, S. 1 (1984)). Die Effizienz des Transfers wurde an Hand der vorgefärbten Markerproteine überprüft.

Die Filter mit den daraufgebundenen Proteinen wurden über Nacht bei Raumtemperatur in 50 ml "Blocking Solution", das ist PBS:
137,0 mM NaCl
2,7 mM KCl
8,0 mM Na₂HPO₄
1,5 mM KH₂PO₄
0,5 mM MgCl₂.6H₂O
1,0 mM CaCl₂.2H₂O
mit 1% BSA, 1% Tween 20 (Polyoxyethylen(20)-sorbitanmonolaurat) und 10% Hitze-inaktiviertem fötalem Kälberserum (HIFKS), gebadet.
Das polyklonale Antiserum gegen VP1 (ATCC VR-1112 AS/GP) wurde vor Verwendung mit einem E. coli - Lysat präinkubiert, um E. coli - spezifische Antikörper zu entfernen. Dazu mischte man gleiche Volumina Antiserum und E. coli-Zellysat, inkubierte 2 Stunden bei Raumtemperatur und über Nacht bei 4°C. Die kreuzreagierenden E. coli Proteine wurden als Immunopräzipitat durch Zentrifugation (5 min, Eppendorfzentrifuge) vom Überstand getrennt. Das Nitrozellulosefilter wurde anschließend 3 Stunden in "Blocking Solution" mit dem polyklonalen Antikörper (präinkubiertes polyklonales Antiserum 1/500 bis 1/1000 in "Blocking Solution" verdünnt) in einer Plexiglasbox auf einer Wippe inkubiert. Danach wurde das Filter unter fließendem Leitungswasser gut gespült (ca. 15 min) und dreimal je 15 min mit 50 ml PBS (+1% Tween 20) gewaschen. Im letzten Schritt wurde das Filter in ca. 50 ml "Blocking Solution" mit den alkalische Phosphatase konjugierten Kaninchen-Anti-Antikörper (1/5000 bis 1/7500 in "Blocking Solution" verdünnt) 3 Stunden bei Raumtemperatur inkubiert. Abschließend wurde das Filter wieder unter fließendem Leitungswasser gut gespült (15 min) und dreimal wie oben mit je 5o ml PBS (+1% Tween 20) gewaschen. Die Anfärbung erfolgte in 10 ml Phosphatasepuffer:
100 mM TrisHCl pH=9,5
100 mM NaCl
5 mM MgCl₂
in Anwesenheit der Farbstoffe Nitro-blue-Tetrazolium (NBT; 165 µg/ml) und 5-Bromo-4-chloro-3-indolyl-Phosphat (BCIP; 82,5 µg/ml). Das alkalische Phosphatase konjugierte Anti-Kaninchen-IgG (Fc), sowie die Farbstoffe NBT und BCIP stammen von Promega Biotec (Protoblot TM-System). Die Färbereaktion wurde ebenfalls durch Spülen unter fließendem Leitungswasser nach ca. 1 min abgestoppt. In Fig. 6 ist ein typisches Bild eines Western blots von pEx34c x 18521 bzw. 18731 wiedergegeben.

### Beispiel 3:

### In vitro Transkription und Translation von pEx34c x 18521 und pEx34c x 18731.

pEx34c x 18521 bzw. 18731 wurden wie oben beschrieben im Großmaßstab in W6(lambda) hochgezüchtet und präpariert. Jeweils 3 µg der zirkulären Plasmide wurden dann pro Ansatz im "Procaryotic DNA directed Translation kit" von Amersham (N 380) eingesetzt. Dieses bakterielle zellfreie System erlaubt die in vitro Expression von Genen die auf einem Plasmid lokalisiert sind, vorausgesetzt, daß die relevanten Kontrollsignale wie die Pribnow-box für die Initiation der Transkription und die Shine-Dalgarno Sequenz für die Translation vorhanden sind. Nachgewiesen werden die exprimierten Produkte durch Einbau von S-35 Methionin. Um die beiden Produkte 18521 und 18731 in vitro zu exprimieren, wurde nach den Inkubationsbedingungen des Herstellers vorgegangen. Als Referenzprobe wurde ein pATI53 Vektor eingesetzt, dessen Expressionsprodukt die β-Lactamase, als Marker auf dem Autoradiogramm verwendet werden kann (Fig. 7, Spur 1).
In Spur 2 und 3 bzw. 4 und 5 ist ein typisches Bild von 18521 bzw. 18731 zu sehen; die hochmolekulare Bande auf dem Autoradiogramm entspricht dem nicht prozessierten 18521 Produkt; die Doppel- bzw. Dreifachbanden dürften auf Abbrüche während der Transkription bzw. Translation zurückzuführen sein. Dieses in vitro System eignet sich sehr gut, um mögliche Inhibitoren gegen P2A auszutesten.

### Beispiel 4:

### In vitro Mutagenese des katalytischen Zentrums von P2A

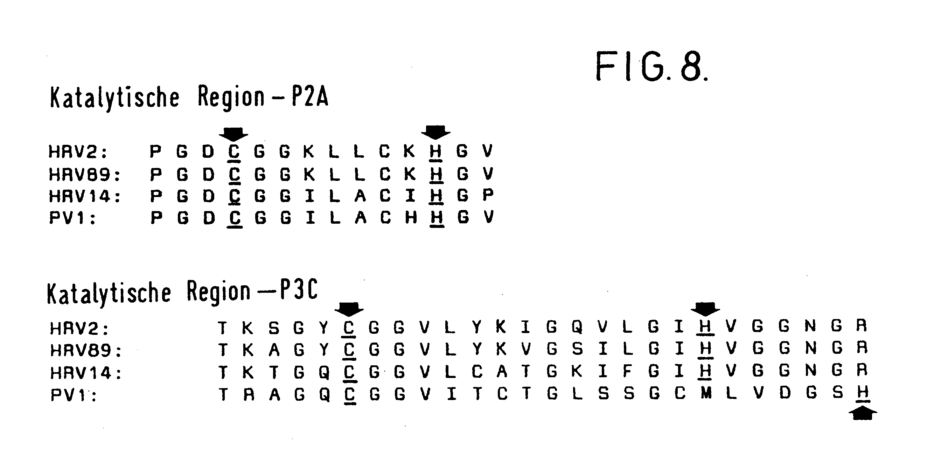

Betrachtet man die Sequenz der P2A-Region einiger Picornaviren und vergleicht diese mit dem katalytischen Zentrum von P3C (zweite viral kodierte Protease), so erkennt man die Konservierung der Aminosäuresequenz dieser Region, insbesondere des wahrscheinlich katalytischen Zentrums (repräsentiert durch Cystein und Histidin). Sowohl bei P2A als auch bei P3C dürfte es sich um eine SH-Protease (Cys....His im aktiven Zentrum) handeln, was der Aminosäuresequenzvergleich in Fig. 8 belegt. Die HRV2-P2A umfaßt 142 Aminosäuren, wobei die an der Katalyse beteiligten Aminosäurereste in Position 114 (Histidin) und 106 (Cystein) bzw. 112 (Cystein) lokalisiert sind (Fig. 15).

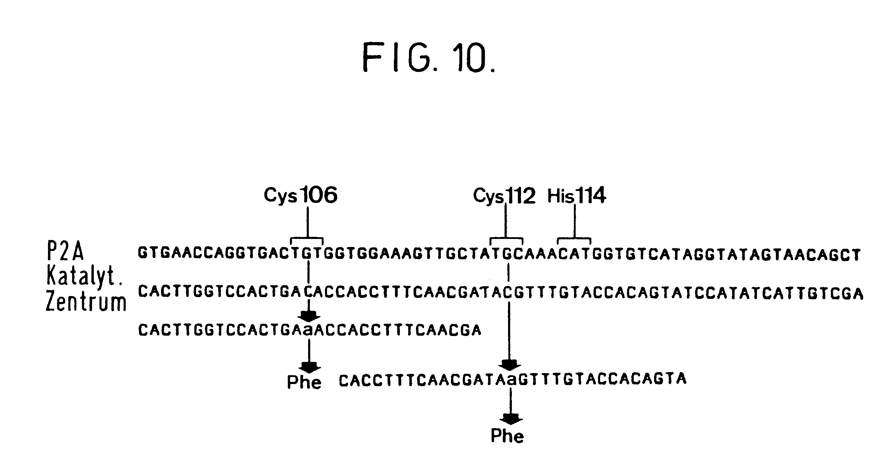

Um nun mehr Information über das katalytische Zentrum zu erhalten, wurden beide in Frage kommenden Cysteine in Position 106 bzw. 112 von HRV2-P2A gegen Phenylalanin durch Oligonukleotid-Mutagenese ausgetauscht. Dazu wurde der Amersham kit "Oligonucleotide-directed in vitro mutagenesis system" verwendet, der auf der Methode von Eckstein et al. (J. W. Taylor, J. Ott und F. Eckstein, 1985, Nucleic Acids Res. 43, 8764-8785) aufgebaut ist. Zunächst mußten dafür 2 Mutationsoligos synthetisiert werden (Applied Biosystems Model 381A DNA Synthesizer; nach Vorschrift des Herstellers), die sowohl das dem Histidin Position 114) näher gelegene Cystein in Position 112 als auch das entferntere Cystein (Position 106) mutagenisieren können (Fig. 10).
Um eine einzelsträngige DNA der P2A-Region zu gewinnen, wurden ca. 10 µg pEx34c x 18521 in 100 µl 1x Eco/Hind-Puffer (50 mM NaCl, 75 mM TrisHCl pH=7,5, 7,5 mM MgCl₂) mit je 50 U an EcoRI und HindIII 4 Stunden bei 37°C verdaut. Das entstandene EcoRI/HindIII-Fragment von 18521 wurde wie oben beschrieben mit DE 81 Papier von einem 1,2% Agarosegel isoliert und in 50 µl TE-Puffer gelöst. Parallel dazu wurden 15 µg M13mp8 in 30 µl 1x Eco/Hind-Puffer mit je 10 U EcoRI und HindIII 2 Stunden bei 37°C verdaut. Mit 0,5 M EDTA pH=8 wurde der Restriktionsenzymverdau gestoppt (Endkonzentration 20 mM), einmal mit Phenol/Chloroform und einmal mit Chloroform extrahiert, mit Ethanol präzipitiert, getrocknet und in 5 µl TE-Puffer aufgenommen.
800 ng EcoRI/HindIII-Fragment von 18521 und 200 ng M13mp8 (EcoRI/HindIII) wurden in 10 µl 1x Ligasepuffer (50 mM TrisHCl pH=7,5, 10 mM MgCl₂, 10 mM DTT, 1 mM ATP und 50 ng/ul BSA) mit 5 U T4-DNA-Ligase bei 14°C über Nacht ligiert. 5 µl des Ligaseansatzes wurden verwendet um 200 µl kompetente JM 101 zu transformieren; dabei und bei allen weiteren Schritten der in vitro Mutagenese wurde nach dem Protokoll des Herstellers (Amersham) vorgegangen, wobei folgende Modifikationen eingeführt wurden:
1.) Anstelle des E. coli Stammes TGl -wie von Amersham empfohlen- wurde JM 101 verwendet, da in den TGl Zellen das Insert (18521) im M13mp8 Vektor nicht stabil war.
2.) Die Übernachtkultur von JM 101 wurde in Minimalmedium hochgezüchtet; das Minimalmedium enthält pro Liter:
   10,5g K₂HPO₄
   4,5g KH₂PO₄
   1,0g (NH₄₎₂SO₄
   0,5g Natriumcitrat x 2H₂O
   0,2g MgSO₄ x 7H₂O
   0,01g Thiamin HCl
   5,0g Glucose; wobei Thiamin HCl und Glucose erst nach dem Autoklavieren der Salzlösung in Form von steril filtrierten Stammlösungen zugesetzt werden.
3.) Um JM 101 Zellen in die log-Phase zu bringen, wurde die Übernachtkultur 1/1000 in 2x TY-Medium verdünnt.

Die beiden Mutationsereignisse für die zwei Cysteine in Position 112 und 106 von HRV2-P2A erbrachten jeweils einige hundert weiße Plaques. Die Mutationseffizienz lag dabei bei über 95%. Je 5 weiße Plaques wurden ausgestochen und in je 1,5 ml 2x TY-Medium mit 20 µl JM 101 (Übernachtkultur im Minimalmedium) transferiert und 6 Stunden bei 37°C unter Schütteln inkubiert. Anschließend wurde 3 min zentrifugiert (Eppendorfzentrifuge) und aus den Zellen wurde der doppelsträngige mutierte M13mp8 x 18521 - Vektor nach der Minipräparationsmethode isoliert. Die isolierten M13mp8 Plasmide wurden in 1x Eco/Hind-Puffer wie oben beschrieben verdaut, auf einem 1,2% Agarosegel aufgetrennt und mit Hilfe von DE 81 Papier wurden die mutierten EcoRI/HindIII-Fragmente von 18521 isoliert. Diese mutierten EcoRI/HindIII-Fragmente wurden in den pEx34c - Vektor zurückligiert, wobei je 250 ng pEx34c (EcoRI/HindIII) und ca. 200 bis 400 ng mutiertes EcoRI/HindIII - Fragment in 20 µl 1x Ligasepuffer und 1 U T4-DNA-Ligase (Boehringer Mannheim) eine halbe Stunde bei Raumtemperatur und über Nacht bei 16°C inkubiert wurden. 10 µl des Ligationsansatzes wurden zur Transformation von kompetenten JM 101 Zellen eingesetzt. Dabei wurden für pEx34c x 18521/106 (entspricht dem Plasmid mit Mutation des Cysteins in Position 106) 15 Klone und für pEx34c x 18521/112 (Mutation des Cysteins in Position 112) 24 Klone erhalten. Jeweils 5 Klone wurden ausgewählt und deren Plasmide im Großmaßstab isoliert. Die gereinigten Plasmide wurden mit HindIII linearisiert und mit 100 U bakterieller alkalischer Phosphatase in 100 mM TrisHCl pH=8 3 Stunden bei 65°C inkubiert. Nach Zugabe von EDTA auf 20 mM wurde zweimal mit Phenol/Chloroform extrahiert und die DNA durch Ethanol gefällt. Die DNA wurde anschließend in 50 µl 50 mM TrisHCl pH=8, 10 mM MgCl₂, 5 mM DTE, mit 25 µCi gamma P-32 ATP (5000 Ci/mmol, Amersham) und 4 U T4-Polynukleotidkinase (BRL) 30 min bei 37°C inkubiert und die markierte DNA mit Ethanol präzipitiert. Unter Zuhilfenahme von EcoRI, das am 5′-Ende des Inserts in pEx34c x 18521/106 bzw. 112 schneidet, wurde die Insert-DNA, die in einem Strang mit P-32 markiert war, erhalten. Die Sequenzierung von 18521/106 und 18521/112 erfolgte nach Maxam und Gilbert (A. Maxam und W. Gilbert, 1980, Methods Enzymol. 65, 499 - 560). Je ein Plasmid von 18521/106 bzw. 112 das die entsprechende mutierte Sequenz im katalytischen Zentrum von P2A aufwies, wurde wie im 2. Beispiel beschrieben im prokaryontischen in vitro Translationssystem von Amersham zur Expression gebracht. Das Plasmid 18521/112, welches das Cystein in unmittelbarer Nähe des Histidinrestes gegen Phenylalanin ausgetauscht vorliegen hat, zeigte dabei eine erhöhte Aktivität von P2A, während die Mutation des vom Histidin weiter entfernt lokalisierten Cysteins zu einer Inhibierung der proteolytischen Aktivität führte (siehe Fig. 11).

### Beispiel 5:

### In vivo Markierung von P2A mit S-35 Methionin

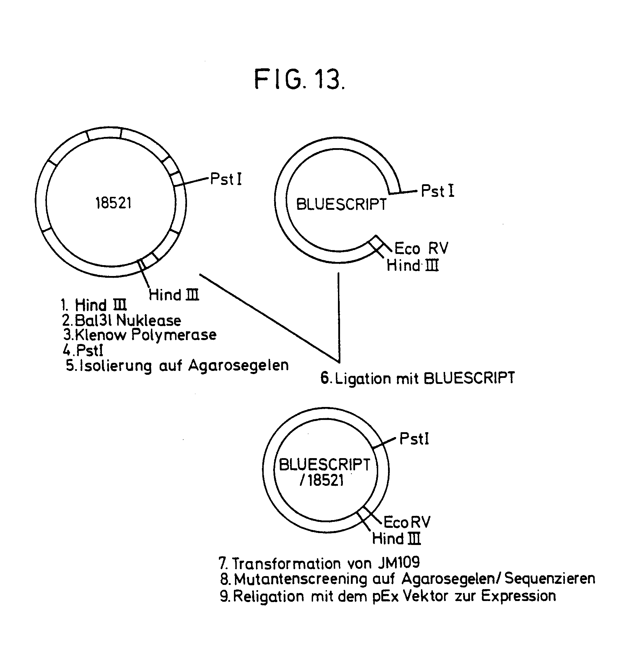

Zur in vivo Markierung von HRV2-P2A wurden 2 verschiedene Expressionsplasmide eingesetzt. Dabei handelt es sich um das oben beschriebene pEx34c x 18521 Expressionssystem und um eine Deletionsmutante von 18521. Diese Deletionsmutante wurde wie folgt hergestellt:
Plasmid-DNA von pEx34c x 18521 wurde nach der Großmaßstabmethode (siehe oben) hergestellt. 300 µg wurden mit dem Restriktionsenzym HindIII (Boehringer Mannheim) in einem Gesamtvolumen von 200 µl verdaut. Je 20 µl dieses Ansatzes wurden bei 30°C 6 - 15 Minuten mit der Exonuklease Bal3l (Biolabs) nach Angaben des Herstellers in einem Volumen von 30 µl inkubiert. Die Nukleasereaktion wurde durch Zugabe von 20 µl einer 0.25 M EDTA Lösung gestoppt und auf Eis gegeben. Nach Extraktion mit Phenol/Chloroform und Chloroform wurde die DNA mit Ethanol ausgefällt und in 10 µl H₂O aufgenommen. Mit dem Klenow Fragment der DNA-Polymerase (Biolabs) und je 2.5 µM der Nukleotide dATP, dCTP, dGTP und dTTP wurden überstehende Enden der Plasmid-DNA aufgefüllt. Die Plasmide wurden wie oben beschrieben extrahiert und ausgefällt. Nach einem weiteren Verdau mit PstI Boehringer Mannheim) wurden die Fragmente auf einem Agarose Gel aufgetrennt und mit Hilfe von DE 81 Papier eluiert. 10 µg des BLUESCRIPT Vektors (Stratagene Cloning Systems) wurden mit EcoRV und PstI verdaut, durch Extraktion gereinigt und ausgefällt. Die isolierten Fragmente, die ein stumpfes Ende, vom Bal3l Verdau und Polymerase Behandlung, und ein überhängendes Ende, vom PstI Verdau, besitzen, wurden in den aufgeschnittenen BLUESCRIPT Vektor hineinligiert (Fig. 13). Kompetente E. coli Stamm JM109 Zellen wurden mit der Ligaselösung transformiert. Transformanten wurden auf Agarplatten mit Ampicillin und X-Gal/IPTG selektiert. Von den Zeitwerten 7, 9, 11, 13 und 15 wurden 109 Deletionsmutanten erhalten. Um die Größe und Lage der Deletionen zu lokalisieren wurde von diesen Klonen Plasmid-DNA nach der Minipräparationsmethode hergestellt, mit PstI und HindIII geschnitten und auf einem Agarose Gel aufgetrennt. Die PstI Stelle blieb durch die Ligation erhalten, während die HindIII Stelle aus dem Polylinker des BLUESCRIPT Vektors stammt und nur 3 Nukleotide nach dem Insert liegt. Durch Größenvergleich mit einem Marker (Lambda DNA mit HindIII verdaut) und untereinander konnten jene Klone identifiziert werden, deren Deletionen nur den C-Terminus von P2A betreffen. 54 Klone wurden nach der Dideoxymethode nach Sanger et al. (Zimmern, et al. Proc. Natl. Acad. Sci. USA, 1978, 75, 4257-4261 ) mit einem Oligonukleotidprimer (Applied Biosystems) sequenziert. 29 Klone, die Deletionen im Bereich des katalytischen Zentrums der Protease P2A und in Richtung C-Terminus von P2A aufwiesen, wurden in den pEx34c Vektor zurückkloniert. Dazu wurden je 1 µg Plasmid-DNA mit PstI und HindIII geschnitten, auf einem Agarose Gel aufgetrennt und die kleineren Fragmente mit DE 81 Papier (siehe Beispiel 1) isoliert. Der Vektor pEx34C x 18521 wurde ebenfalls mit diesen Enzymen verdaut und das größere Fragment aus dem Gel isoliert. Nach Ligation der Deletionsfragmente mit dem Vektorfragment wurden kompetente E. coli, Stamm 537, transformiert. Die Expression und der Nachweis der Fusionsproteine erfolgte durch Western blot Analyse mit einem polyklonalen Serum gegen VP1. Dabei zeigte der Klon 13A, dessen C-Terminus von P2A um 10 Aminosäuren verkürzt worden war (Deletionsmutante endet mit Nukleotidnummer 3555), keine proteolytische Aktivität mehr, während eine zweite Deletionsmutante 13W, der 6 Aminosäuren vom P2A-C-Terminus fehlten (endet mit Nukleotidnummer 3569) proteolytische Aktivität aufwies. Das bedeutet, daß der C-terminale Bereich von P2A um mindestens 6 Aminosäuren verkürzt werden kann, ohne das proteolytische Processing zu beeinflussen. Die Deletion zwischen der zehnten und sechsten Aminosäure des P2A-C-Terminus führt offensichtlich zur Zerstörung eines essentiellen Abschnitts von P2A (s. Fig. 14).

Eine weitere Deletionsmutante von 18521, die keine proteolytische Funktion von P2A aufwies, endet mit der für HRV2 kodierenden Region 18 Aminosäuren vor dem Carboxyterminus von P2A. Diese Mutante wurde 13L genannt (Fig. 14).
Die beiden Expressionsplasmide 18521 und 13L in E. coli, Stamm 537 wurden wie in Beispiel 1 angeführt über Nacht bei 28°C hochgezüchtet. Anschließend wurden 100 µl der Zellsuspension in 5 ml M9-Medium:
10 ml 10x M9-Salze (siehe Maniatis, T. loc. cit.)
0,5 ml 1M MgSO₄
1 ml 20% Glucose
0,2 ml Thiaminlösung (20 mg/ml)
1 ml Biotinlösing (0.2 mg/ml)
0,1 ml Aminosäurelösung von Ile, His, Val, Thr und Leu; je 20 mg/ml 0,01 ml 1M CaCl2 auf 100 ml aufgefüllt.
auf OD600 von 0,15 verdünnt (das entspricht etwa 100 ul Zell-suspension in 5 ml M9-Medium. Die Kultur wurde dann bei 28°C bis zu einer OD600 von 0,3 hochgezüchtet (ca. 2 Stunden bei 28° C). Die Expression der unter der Kontrolle des linken lambda-Promoter stehenden HRV2-Sequenzen, erfolgte wie in Beispiel 1 beschrieben (2 Stunden bei 42°C). Nach der induzierten Expression wurden 1 ml der Zellsuspension entnommen, kurz zentrifugiert (30 sek in der Eppendorfzentrifuge) und in 100 µl M9-Medium (+ 100 µCi S-35 Methionin) aufgenommen. Die Zellen wurden kurz (3 min bei 42°C) inkubiert und die Reaktion mit kaltem PBS gestoppt. Die Zellen wurden kurz zentrifugiert und in 1 ml PBS resuspendiert, um die überschüssige Radioaktivität zu entfernen. Die wie in Beispiel 1 sonikierten Proben wurden 3 min zentrifugiert und das Pellet in 50 µl Lämmli-Sample-puffer gelöst. Der Überstand der Zellysate wurde mit 200 µl 50% TCA versetzt, 30 min bei 4°C inkubiert, 10 min zentrifugiert und das Pellet in 50 µl Lämmli-Sample-puffer gelöst. Je 10 µl der Proben wurden auf einem 12,5% SDS-PAA (Dicke 0,75mm) aufgetrennt. In Fig. 12 ist das Autoradiogramm dieses Experimentes zu sehen. Sowohl im Überstand als auch im Pellet des Expressionsystems von 18521 ist eine spezifische Bande zu erkennen, die P2A (plus einem Teil von P2B) repräsentiert. Diese Bande ist im Expressionssystem der Deletionsmutante 13L nicht anzutreffen (siehe Fig. 12).

### Beispiel 6:

Identifikation einer essentiellen Aminosäure (Arg 134) im C-Terminus der Protease 2A.

Betrachtet man die Aminosäuresequenz von 2A und ihre innerhalb der Rhino-, Polio- und Coxsackieviren hochkonservierten Aminosäuren (siehe Fig. 15), so erkennt man, daß innerhalb der letzten 6 und 10 Aminosäuren vom C-Terminus von 2A nur das Arginin in Position 134 konserviert ist. Um nun herauszufinden, ob dieser Rest eine fundamentale Bedeutung in der Katalyse besitzt, wurde das Arginin 134 durch in vitro Mutagenese in ein Glutamin umgewandelt. Die Mutagenese wurde an einem 1,3 kb Pst I/Hind III Fragment von pEx18521 (entspricht pEx34cx18521), welches in die Pst I/Hind III Stelle von BLUESCRIPT subkloniert worden war, durchgeführt. Diese, von einem Oligonukleotid gesteuerte in vitro Mutagenese, wurde mit Hilfe eines Amersham kits (Oligonucleotide-directed in vitro mutagenesis) nach der Vorschrift des Herstellers durchgeführt. Drei positive Klone wurden, wie oben beschrieben, sequenziert. Das mutierte Pst I/Hind III Fragment wurde dazu verwendet, um das Wildtyp Pst I/Hind III Fragment in pEx18521 zu ersetzen. Der mutierte Expressionsvektor pEx18521[Arg134-->Gln] wurde, wie in Beispiel 2 beschrieben, zur Expression gebracht und das Muster der Expressionsprodukte auf einem Proteingel und mit Hilfe eines Western blot analysiert (siehe Fig. 16). Die Mutation des Arginin 134 zu Glutamin führt zum Verschwinden der proteolytischen Aktivität; es wird ausschließlich das unprozessierte 75K Protein gebildet.

### Beispiel 7:

### Produktion des Peptidantikörpers PC20

Die letzten 20 Aminosäuren der Protease 2A stellen eine potentielle antigene Determinante dar. Ein Peptid (PC20), welches genau diese Aminosäuresequenz aufwies, wurde wie in Beispiel 9 beschrieben synthetisiert und zur Induktion von Antikörpern in Kaninchen verwendet: 570 µg dieses Peptids wurden in 0,4 ml PBS Lösung aufgenommen und in einer 5 ml Spritze aufgezogen. In einer zweiten 5 ml Spritze wurden 0,5 ml Freund'sches Adjuvans (CAF; GIBCO) aufgezogen und die beiden Komponenten wurden anschließend mit Hilfe eines Dreiweg-Sperrhahnventils vermischt, bis eine Emulsion gebildet wurde. Das Kaninchen wurde im Ohr arteriell punktiert, um ein Pre-Serum für die Negativkontrolle zu erhalten. Die Immunisierung erfolgte durch subkutane Injektion der Peptid/CFA Mischung an 4 verschiedenen Stellen (0,2 ml/Injektionsstelle) des Rückenbereiches. Nach 5 Wochen wurde mit 1,2 ml Peptidlösung durch Injektion von jeweils 0,2 ml intramuskulär in den Rückenteil "geboostet". Acht Tage später wurde das Blut durch Herzpunktion entnommen. Das Blut ließ man bei Zimmertemperatur gerinnen, Fibrin und geformte Bestandteile wurden mit einem sterilen Stäbchen entfernt und das Blut bei 2000 rpm zentrifugiert. Das Serum wurde aliquotisiert und bei -18°C gelagert.

Die Überprüfung des Hydrophobizitätsprofils der Protease 2A nach Kyte und Doolittle (Kyte,J. and Doolittle, R.F. (1983) J. Mol. Biol. 157, 105-132) und die Sekundärstrukturanalyse dieser Region (Pallai, P.V., Mabila, M., Goodman, M., Vale, W. and Rivier, J. (1983) J. Am. Chem. Soc. 85, 2149 - 2154 ) wiesen darauf hin, daß die letzten 20 Aminosäuren der Protease 2A eine potentielle antigene Determinante darstellen. Ein Peptid (PC20), das genau diese Aminosäuresequenz aufwies, wurde daher synthetisiert, beispielsweise der Vorschrift aus Beispiel 9 folgend und zur Induktion von Antikörpern in Kaninchen verwendet. Ein Kaninchen wurde im Ohr arteriell punktiert, um ein Pre-Serum für die Negativkontrolle zu erhalten. Die Immunisierung erfolgte durch subkutane Injektion einer Peptid/CFA Mischung an 4 verschiedenen Stellen des Rückenbereiches. Das gewonnene Serum wurde aliquotisiert und bei -18°C gelagert.

### Beispiel 8:

Etablierung eines Expressionssystems zur Darstellung nativer Protease 2A, sowie Analyse von Punktmutanten in dem wahrscheinlich aktiven Zentrum von 2A.

Um die Rolle einiger hochkonservierter Aminosäuren in der Region des wahrscheinlich aktiven Zentrums zu untersuchen (siehe Fig. 15), wurde unter Verwendung einer Oligonukleotidkassette die in vitro Mutagenese und die Deletion einzelner Aminosäuren in dieser Region durchgeführt. Parallel dazu wurde diese Methode verwendet, um native Protease 2A zu exprimieren. Ausgehend von pEx18521 wurde durch Verdau mit Apa I (Nukleotidnummer 3458 der HRV2 cDNA) und Hind III (Restriktionsschnittstelle stammt aus der Polylinkerregion des Vektors; siehe Beispiel 1) nach Vorschrift des Herstellers (Biolabs) ein 264 bp langes DNA-Fragment gewonnen, welches durch zwei doppelsträngige Oligonukleotide WT 12 und WT 34 mit Apa I/Hind III "sticky ends" ersetzt wurde (siehe Fig. 17). Zunächst wurde je 1 µg der einzelsträngigen Oligonukleotide WT1, WT2, WT3 und WT4 in jeweils 10 µl (20 mM TrisHCl PH=7,5, 10 mM MgCl2, 20 mM DTT und 1 mM ATP) mit je 2 U T4-Polynukleotidkinase (Biolabs) 30 min bei 37°C kinasiert. Anschließend wurden die Kinaseansätze von WT1 und WT2, bzw. von WT3 und WT4 vereinigt, 10 min bei 68°C, 30 min bei 45°C, 10 min bei Raumtemperatur und anschließend kurz auf Eis inkubiert. Die dermaßen kinasierten und hybridisierten Oligonukleotide wurden vereinigt, mit einer 10 mM ATP Lösung wurde die Konzentration auf 1 mM eingestellt und für die Ligation mit 28 U T4-DNA-Ligase (Boehringer Mannheim) versetzt. Die Ligation selbst wurde zunächst 2 Stunden bei 20°C durchgeführt, dann wurden nochmals 7 U T4-DNA-Ligase zugesetzt und der Ligationsansatz 40 Stunden bei 14°C inkubiert. Anschließend wurde der Ligationsansatz bei 70°C 10 min inkubiert, mit einer 1M NaCl-Lösung auf 100 mM gebracht und mit 50 U Apa I und 20 U Hind III wurden die entstandenen multimeren Formen des Oligonukleotids "nachgeschnitten". Die Reinigung und Isolierung des korrekten, doppelsträngigen Apa I/Hind III Fragmentes, sowie die Rückklonierung dieses Fragmentes in pEx18521 (mit Apa I und Hind III geschnitten) und die Identifikation mittels Sequenzierung wurde, wie in Beispiel 1 beschrieben, durchgeführt. Ein positiver Klon wurde ausgewählt und zu Expressionsstudien sowie zu Transaktivitätstests herangezogen. Die Induktion der Expression erfolgte genau wie in Beispiel 2 beschrieben und erbrachte das in Fig. 18 gezeigte Ergebnis.

### Beispiel 9:

### in vitro Transaktivitätstest für die Protease 2A unter Verwendung eines Spaltstellenpeptids

Die in diesem Beispiel verwendeten Peptide wurden nach der "solid phase" Methode (Merrifield, R.B. (1963) J. Am. Chem. Soc., 85, 2149 - 2154) synthetisiert. Anschließend wurden die Peptide über "reverse phase" HPLC gereinigt (0,1% Trifluoressigsäure und Acetonitril als mobile Phase) und mit Hilfe von HPLC Analyse, "fast atom bombardment mass spectrometry (FAB-MS) und Aminosäuresequenzierung (Hunkapiller, M.W. and Hood, L.E., loc. cit.) identifiziert.
Wie in Beispiel 2 beschrieben wurde die Expression der 2A Expressionssysteme (siehe Beispiel 8) im E. coli Stamm 537 induziert. Nach 2 Stunden Inkubation bei 42°C wurden die Zellen von 1 ml der Kultur geerntet (2 min Eppendorfzentrifuge, 4°C) und in 500 µl HEPES Puffer (100 mM NaCl, 10 mM HEPES pH=7,4, 1 mM EDTA und 1 mM DTT) resuspendiert. Das Aufbrechen der Zellen wurde mit Hilfe eines M.S.E. Ultrasonic Power Gerätes vorgenommen 3 mal 30 sek; im Eiswasserbad), wobei zwischen den einzelnen Sonizierungen eine Pause von 30 sek eingeschaltet wurde, um ein Überhitzen der Proben zu vermeiden. Unlösliches Material wurde durch Zentrifugation (2 min Eppendorfzentrifuge) entfernt und jeweils 100 µl vom Überstand wurden mit 5 µl einer wässerigen Peptidlösung von Ac-TRPIITTAGPSDMYVH (4 mg/ml) versetzt und bei 37°C 20 min lang inkubiert. Die Reaktion wurde durch Zugabe des gleichen Volumens an 1 M HClO₄ abgestoppt. Die Proben wurden anschließend 20 min auf Eis inkubiert, 10 min bei 4°C zentrifugiert (Eppendorfzentrifuge), mit gleichem Volumen 1,4 M K₂HPO₄ versetzt, 5 min bei Raumtemperatur inkubiert und das entstandene Präzipitat durch Zentrifugation entfernt (5 min, Eppendorfzentrifuge). Der Peptid enthaltende Überstand wurde anschließend über "reverse phase" HPLC aufgetrennt, wobei eine 0,1% Trifluoressigsäure und Acetonitril als mobile Phase eingesetzt wurden.

Das ursprüngliche 16 Aminosäuren lange Peptid-Substrat und ein Referenzpeptid, welches das C-terminale Spaltprodukt (GPSDMYVH) repräsentiert, wurden wie oben beschrieben ebenfalls auf der HPLC Säule aufgetrennt und N-terminal sequenziert wie oben beschrieben. Figur 19 zeigt die HPLC Profile der Peptide nach Inkubation des Peptid-Substrates in verschiedenen bakteriellen Extrakten.

## Patentansprüche

1. Expressionssystem aus einem Plasmidanteil und einem Insert, dadurch gekennzeichnet, daß der Plasmidanteil in der Lage ist, ein von dem Insert kodiertes Fusionsprotein effektiv zu exprimieren und daß das Insert für ein Fusionsprotein aus einem enzymatisch aktiven Anteil einer rhinoviralen 2A Protease und einem von diesem autokatalytisch abspaltbaren Polypeptidanteil kodiert.

2. Expressionssystem nach Anspruch 1, dadurch gekennzeichnet, daß der Plasmidanteil eine prokaryontische ribosomale Bindungsstelle, eine kodierende Region für einen die Stabilität des exprimierten, von dem Insert kodierten Fusionsproteins in der Zelle erhöhenden Fusionsanteils, einen das Fusionsprotein kontrollierenden Promotor, eine Polylinkerregion in drei verschiedenen Leserahmen, vorzugsweise mit Schnittstellen für EcoRI, BamHI, HindIII, PstI, BglII und XbaI und die "ori"- und Ampicillin-resistenz-region von pBR322 enthält.

3. Expressionssystem nach Anspruch 1, dadurch gekennzeichnet, daß der enzymatisch aktive Anteil P2A-(P2B) von HRV2 ist.

4. Expressionssystem nach Anspruch 1, dadurch gekennzeichnet, daß der enzymatisch aktive Anteil die HRV2-P2A Protease ist.

5. Expressionssystem nach Anspruch 1, dadurch gekennzeichnet, daß der abspaltbare Polypeptidanteil ein rhinovirales Protein, bevorzugt ein rhinovirales Protein VP1, besonders bevorzugt (VP3)-VP1 von HRV2, insbesondere das virale Protein HRV2-VP1 oder Teilen von diesen ist.

6. Expressionssystem nach Anspruch 1, dadurch gekennzeichnet, daß das Insert für (VP3)-VP1-P2A-(P2B) von HRV2 oder für (VP3)-VP1-P2A von HRV2 kodiert oder die Nukleotide 2145-3698 der cDNA von HRV2 oder die Nukleotide 2145-3585 der cDNA von HRV2 enthält.

7. Expressionssystem nach einem der Ansprüche 1 bis 6, dadurch gekennzeichnet, daß das Insert ein DNA-Molekül nach einem der Ansprüche 1 bis 6 im richtigen Leserahmen ist.

8. Verwendung des Expressionssystems nach einem der Ansprüche 1 bis 7 als Testsystem für Inhibitoren rhinoviraler Proteasen.

9. Verwendung des Expressionsystems nach einem der Ansprüche 1 bis 8, als Testsystem für P2-A-Inhibitoren, bevorzugt für HRV2-P2A-Inhibitoren.

10. "Trans-assay" als Testsystem für rhinovirale Inhibitoren, dadurch gekennzeichnet, daß er das Expressionssystem nach einem der Ansprüche 1 bis 7 enthält.

11. Verfahren zur effektiven Expression einer rhinoviralen 2A Protease, dadurch gekennzeichnet, daß
a. ein Insert, das für ein Fusionsprotein aus einem enzymatisch aktiven Anteil, der eine rhinovirale 2A Protease darstellt, und einem von diesem Anteil autokatalytisch abspaltbaren Polypeptidanteil kodiert, in einen Expressionsvektor kloniert,
b. in einen geeigneten Wirtsorganismus transformiert und
c. das Fusionsprotein in dem Wirtsorganismus zur Expression gebracht wird.

## Claims

1. Expression system consisting of a plasmid component and an insert, characterised in that the plasmid component is capable of effectively expressing a fusion protein coded by the insert and the insert codes for a fusion protein consisting of an enzymatically active component of a rhinoviral 2A protease and a polypeptide component capable of being autocatalytically cleaved therefrom.

2. Expression system according to claim 1, characterized in that the plasmid component contains a prokaryotic ribosomal binding site, a coding region for a fusion component which increases the stability of the expressed fusion protein in the cell coded by the insert, a promoter controlling the fusion protein, a polylinker region in three different reading frames, preferably with cutting sites for EcoRI, BamHI, HindIII, PstI, BglII and XbaI and the "ori" and ampicillin-resistance region of pBR322.

3. Expression system according to claim 1, characterised in that the enzymatically active component is P2A-(P2B) from HRV2.

4. Expression system according to claim 1, characterised in that the enzymatically active component is the HRV2-P2A-protease.

5. Expression system according to claim 1, characterised in that the cleavable polypeptide component is a rhinoviral protein, preferably a rhino-viral protein VP1, more preferably (VP3) - VP1 of HRV2, more particularly the viral protein HRV2-VP1 or parts thereof.

6. Expression system according to claim 1, characterised in that the insert codes for (VP3) - VP1-P2A-(P2B) of HRV2 or for (VP3) - VP1-P2A of HRV2 or contains the nucleotides 2145 to 3698 of the cDNA of HRV2 or the nucleotides 2145 to 3585 of the cDNA of HRV2.

7. Expression system according to one of claims 1 to 6, characterized in that the insert is a DNA molecule according to one of claims 1 to 6 in the correct reading frame.

8. Use of the expression system according to one of claims 1 to 7 as a test system for inhibitors of rhino-viral proteases.

9. Use of the expression system according to one of claims 1 to 8, as a test system for P2A inhibitors, preferably for HRV2 P2A inhibitors.

10. "Trans-assay" as a test system for rhinoviral inhibitors, characterized in that it contains the expression system according to one of claims 1 to 7.

11. Process for effective expression of a rhinoviral 2A protease, characterized in that
a. an insert which codes for a fusion protein from an enzymatically active component constituting a rhino viral 2A protease, and a polypeptide component which can be cleaved autocatalytically from this component, is cloned into an expression vector,
b. transformed into a suitable host organism and
c. the fusion protein is expressed in the host organism.

## Revendications

1. Système d'expression constitué par une partie plasmidique et un insert, caractérisé en ce que la partie plasmidique est capable d'exprimer de manière efficace une protéine de fusion codée par l'insert et en ce que l'insert code une protéine de fusion constituée par une partie active du point de vue enzymatique d'une protéase rhinovirale 2A et par une partie polypeptidique clivable de manière autocatalytique de la précédente.

2. Système d'expression selon la revendication 1, caractérisé en ce que la partie plasmidique contient un site de fixation ribosomique procaryotique, une région codante pour une partie de fusion augmentant la stabilité dans la cellule de la protéine de fusion codée par l'insert et exprimée, un promoteur contrôlant la protéine de fusion, une région de lieur multisite dans trois cadres de lecture différents, de préférence avec des sites de coupure pour EcoRI, BamHI, HindIII, PstI, BglII et XbaI et la région "ori" et de résistance à l'ampicilline de pBR322.

3. Système d'expression selon la revendication 1, caractérisé en ce que la partie active du point de vue enzymatique est p2A-(P2B) de HRV2.

4. Système d'expression selon la revendication 1, caractérisé en ce que la partie active du point de vue enzymatique est la protéase HRV2-P2A.

5. Système d'expression selon la revendication 1, caractérisé en ce que la partie polypeptidique clivable est une protéine rhinovirale, de préférence une protéine rhinovirale VP1, de préférence encore (VP3)-VP1 de HRV2, en particulier la protéine virale HRV2-VP1 ou des parties de celles-ci.

6. Système d'expression selon la revendication 1, caractérisé en ce que l'insert code (VP3)-VP1-P2A-(P2B) de HRV2 ou (VP3)-VP1-P2A de HRV2 ou contient les nucléotides 2145-3698 de l'ADNc de HRV2 ou les nucléotides 2145-3585 de l'ADNc de HRV2.

7. Système d'expression selon l'une des revendications 1 à 6, caractérisé en ce que l'insert est une molécule d'ADN selon l'une des revendications 1 à 6 dans le cadre de lecture correct.

8. Utilisation du système d'expression selon l'une des revendications 1 à 7 comme système de test pour inhibiteurs des protéases rhinovirales.

9. Utilisation du système d'expression selon l'une des revendications 1 à 8 comme système de test pour inhibiteurs de P2A, de préférence pour innibiteurs de P2A de HRV2.

10. "Test trans" en tant que système de test pour inhibiteurs rhinoviraux, caractérisé en ce qu'il contient le système d'expression selon l'une des revendications 1 à 7.

11. Procédé d'expression efficace d'une protéase rhinovirale 2A, caractérisé en ce que
a. un insert qui code une protéine de fusion constituée par une partie active du point de vue enzymatique qui représente une protéase rhinovirale 2A et par une partie polypeptidique clivable de manière autocatalytique de cette partie est cloné dans un vecteur d'expression,
b. est transformé dans un organisme hôte approprié et
c. la protéine de fusion est amenée à être exprimée dans l'organisme hôte.
